# EUROPEAN PATENT APPLICATION

(11) **EP 4 620 972 A1**
(43) Date of publication of application: **24.09.2025**
(21) Application number: 23892091.2
(22) Date of filing: 20.11.2023
(51) Int. Cl.: C07K 14/705, C07K 14/725, C07K 16/28, C12N 5/0783, A61K 39/00, A61K 35/17, A61P 35/00

(54) **DUAL GENE-TRANSDUCED IMMUNE CELL USING CTLA-4 VARIANT AND CHIMERIC ANTIGEN RECEPTOR AND USE THEREOF**

(30) Priority: 18.11.2022 KR 20220155885
(71) Applicant: Seoul National University R&DB Foundation, Seoul 08826 (KR); Ticaros Co., Ltd, Seoul 04784 (KR)
(72) Inventor: CHOI, Kyung Ho, Seoul 02814 (KR); LEE, Ji Eun, Seoul 03115 (KR); LEE, Seo Ho, Seoul 06196 (KR); PARK, Hyung Bae, Seoul 02562 (KR)
(74) Representative: Schiweck Weinzierl Koch Patentanwälte Partnerschaft mbB
(86) International application number: PCT/KR2023/018677
(87) International publication number: WO 2024/107029

(57) **Abstract**

The present invention relates to a dual gene-engineered T cell using a CTLA-4 mutant and a chimeric antigen receptor, and a composition for anti-cancer immunotherapy comprising the same. According to one aspect, the dual gene-engineered T cell using a CTLA-4 mutant and a chimeric antigen receptor, and the composition for anti-cancer immunotherapy comprising the same can be advantageously used in immune cell therapy for the treatment of cancer as the introduction of the CTLA-4 mutant induces enhancement of T cell function without accompanying side effects such as autoimmune diseases, etc., whereby improved tumor treatment efficiency and suppression of tumor recurrence can be achieved through the injection of a small number of T cells.

## Description

### [Technical Field]

The present invention relates to a dual gene-engineered immune cell using a CTLA-4 mutant and a chimeric antigen receptor (CAR), and uses thereof, wherein expression of a CTLA4-CD28 fusion protein together with a chimeric antigen receptor results in enhanced tumor specificity and antitumor effect, and can be effectively used for anti-cancer immunotherapy.

### [Background Art]

Classical anti-cancer T cell therapy has shown promise as a useful immune cell therapy in early clinical trials, but difficulties in obtaining sufficient numbers of tumor-specific T cells and the need for enhanced efficacy have been raised. Blocking antibodies against the T cell inhibitory receptor CTLA have been marketed under FDA approval to enhance the efficacy of tumor-specific T cells in patients. However, autoimmune side effects due to activation of self-antigen-specific T cells are a problem with systemic administration.

In previous research, the present inventors designed a CTLA4 function-inhibiting mutant called CTLA4-CD28 chimera (CTC28) molecule to enhance the efficacy of tumor-specific T cell therapy, and proposed an efficacy-enhanced T cell therapy in which CTLA4 function is blocked without autoimmune side effects through the production of CTC28 gene-transduced T cells (Patent Document 0001). However, obtaining sufficient numbers of tumor-specific T cells still remained a challenge to be solved.

A chimeric antigen receptor is a recombinant receptor that links an antibody portion that binds to a tumor surface antigen to an activation signal portion inside a T cell. When the CAR gene is transduced into a patient's peripheral blood T cells, it has the advantage of being able to produce a large number of tumor-specific T cells (CAR T cells) in a short period of time. In this invention, taking advantage of the fact that multiple genes can be loaded into a single retrovirus or lentivirus vector, T cells transduced with both CAR and CTC28 genes were produced to develop CAR T cells that achieve both production of large numbers of tumor-specific CAR T cells and functional enhancement with a single gene introduction.

### [Prior Art Reference]

### [Patent Reference].

(Patent reference 1) Korean Patent Laid-open Publication No. 2013-0045284 (publication date: May 6, 2013)

### [Detailed Description of the Invention]

### [Technical Problem]

The present inventors confirmed that unlike second generation CAR molecules containing the CD28 intracellular domain, first generation CAR molecules or second generation CAR molecules containing the 4-1BB intracellular domain showed synergistically enhanced tumor specificity and anti-tumor effects when co-expressed with CTC28, and completed the invention of CTC28-loaded CAR T cells.

### [Technical Solution]

One aspect provides a construct comprising:
(i) a first gene encoding a fusion protein (CTLA4-CD28 fusion protein) comprising a CTLA4 (Cytotoxic T Lymphocyte Antigen-4) protein or a domain thereof; and CD28 or a domain thereof; and
(ii) a second gene encoding a chimeric antigen receptor (CAR) comprising an antigen binding domain; an extracellular spacer domain; a transmembrane domain; and an intracellular signaling domain.

Another aspect provides a vector comprising the construct.

Another aspect provides an immune cell into which the construct or a vector comprising the construct has been introduced.

Another aspect provides an immune cell comprising a protein expressed from the construct.

Another aspect provides an immune cell comprising:
(i) a first gene encoding a fusion protein (CTLA4-CD28 fusion protein) comprising a CTLA4 protein or a domain thereof; and CD28 or a domain thereof; and
(ii) a second gene encoding a chimeric antigen receptor comprising an antigen binding domain; an extracellular spacer domain; a transmembrane domain; and an intracellular signaling domain.

Another aspect provides an immune cell expressing both:
(i) a fusion protein (CTLA4-CD28 fusion protein) comprising a CTLA4 protein or a domain thereof; and CD28 or a domain thereof; and
(ii) a chimeric antigen receptor comprising an antigen binding domain; an extracellular spacer domain; a transmembrane domain; and an intracellular signaling domain.

Another aspect provides a composition comprising the construct, the vector, or the immune cell.

Another aspect provides a pharmaceutical composition for the prevention or treatment of a disease (e.g., cancer), comprising the construct, the vector, or the immune cell and a pharmaceutically acceptable carrier.

Another aspect provides the use of the construct, the vector, the immune cell, or the composition for the prevention or treatment of a disease (e.g., cancer).

Another aspect provides the use of the construct, the vector, the immune cell, or the composition for the manufacture of a medicament for the prevention or treatment of a disease (e.g., cancer).

Another aspect provides a method for the prevention or treatment of a disease (e.g., cancer), comprising the step of administering the construct, the vector, the immune cell, or the composition to a subject in need thereof.

Another aspect provides a method for producing an immune cell expressing the CTLA4-CD28 fusion protein and the chimeric antigen receptor, comprising the step of introducing the first gene and the second gene into the immune cell.

Another aspect provides a method for producing an immune cell expressing the CTLA4-CD28 fusion protein and the chimeric antigen receptor, comprising the step of introducing the construct or the vector into the immune cell.

### [Effect of Invention]

According to one aspect, the dual gene-engineered T cell using a CTLA-4 mutant and a chimeric antigen receptor, and the composition for anti-cancer immunotherapy comprising the same can be useful in immune cell therapy for the treatment of cancer as the introduction of the CTLA-4 mutant induces enhancement of T cell function without accompanying side effects such as autoimmune diseases, etc., whereby improved tumor treatment efficiency and suppression of tumor recurrence can be achieved through the injection of a small number of T cells.

### [Brief Description of Drawings]

FIG. 1 is a schematic diagram showing the dual gene-engineered T cell using the CTLA-4 mutant and the chimeric antigen receptor.
FIG. 2 shows the gene construct structures of the 1^{st} generation CAR (mCD19z) and the 1^{st} generation CAR and CTLA4-CD28 (mCD19z-CTC28).
FIG. 3 shows graphs analyzing the expression of CAR and CTLA4 proteins on the surface of T cells transduced with the 1^{st} generation CAR (mCD19z) or the 1^{st} generation CAR and CTC28 (mCD19z-CTC28).
FIG. 4 shows a graph analyzing the tumor killing ability of T cells transduced with the 1^{st} generation CAR (mCD19z) or the 1^{st} generation CAR and CTC28 (mCD19z-CTC28) against A20 cells.
FIG. 5 illustrates a graph showing the amount of IFN-γ secreted by T cells transduced with the 1^{st} generation CAR (mCD19z) or the 1^{st} generation CAR and CTC28 (mCD19z-CTC28) when co-cultured with A20 cells.
FIG. 6 illustrates graphs showing the changes in tumor size in mice injected with T cells transduced with the 1^{st} generation CAR (mCD19z) or the 1^{st} generation CAR and CTC28 (mCD19z-CTC28).
FIG. 7 shows the gene construct structures of the CD28-based 2^{nd} generation CAR (mCD1928z) and the 2^{nd} generation CAR and CTLA4-CD28 (mCD1928z-CTC28).
FIG. 8 shows graphs analyzing the expression of CAR and CTLA4 proteins on the surface of T cells transduced with the CD28-based 2^{nd} generation CAR (mCD1928z) or the 2^{nd} generation CAR and CTC28 (mCD1928z-CTC28).
FIG. 9 illustrates a graph showing the amount of IFN-γ secreted by T cells transduced with the CD28-based 2^{nd} generation CAR (mCD1928z) or the 2^{nd} generation CAR and CTC28 (mCD1928z-CTC28) when co-cultured with A20 cells.
FIG. 10 illustrates a graph showing the changes in tumor size in mice injected with T cells transduced with the CD28-based 2^{nd} generation CAR (mCD1928z) or the 2^{nd} generation CAR and CTC28 (mCD1928z-CTC28).
FIG. 11 shows the gene construct structure of the 41BB-based 2^{nd} generation CAR (mCD19BBz) and the 2^{nd} generation CAR and CTLA4-CD28 (mCD19BBz-CTC28).
FIG. 12 illustrates graphs analyzing the expression of CAR and CTLA4 proteins on the surface of T cells transduced with the 41BB-based 2^{nd} generation CAR (mCD19BBz) or the 2^{nd} generation CAR and CTC28 (mCD19BBz-CTC28).
FIG. 13 illustrates a graph showing the amount of IFN-γ secreted by T cells transduced with the 41BB-based 2^{nd} generation CAR (mCD19BBz) or the 2^{nd} generation CAR and CTC28 (mCD19BBz-CTC28) when co-cultured with A20 cells.
FIG. 14 illustrates graphs showing the changes in tumor size in mice injected with T cells transduced with the 41BB-based 2^{nd} generation CAR (mCD19BBz) or the 2^{nd} generation CAR and CTC28 (mCD19BBz-CTC28) (Tumor size = (long axis)² x short axis/2).
FIG. 15 shows the gene construct structures of the 2^{nd} generation CAR (mCD19BBz) and the 2^{nd} generation CAR and CTLA4-CD28 (mCD19BBz-CTC28) containing the T cell tracking marker (Thy1.1).
FIG. 16 shows graphs analyzing the expression of Thy1.1 and CTC28 proteins on the surface of CAR T cells transduced with mCD19BBz (CD19) or mCD19BBz-CTC28 (CD19-CTC28).
FIG. 17 shows graphs analyzing the tumor killing ability of CAR T cells transduced with mCD19BBz (CD19) or mCD19BBz-CTC28 (CD19-CTC28) against A20 cells.
FIG. 18 illustrates graphs showing the amount of IFN-γ, IL-2, and TNF-α secreted by CAR T cells transduced with mCD19BBz (CD19) or mCD19BBz-CTC28 (CD19-CTC28) when co-cultured with A20 cells.
FIG. 19 shows graphs analyzing the changes in the proliferative capacity of CD8 CAR T cells transduced with mCD19BBz (CD19) or mCD19BBz-CTC28 (CD19-CTC28).
FIG. 20 illustrates graphs showing the changes in tumor size in mice injected with CAR T cells transduced with mCD19BBz (CD19) or mCD19BBz-CTC28 (CD19-CTC28).
FIG. 21 illustrates graphs showing the survival rate of mice injected with CAR T cells transduced with mCD19BBz (CD19) or mCD19BBz-CTC28 (CD19-CTC28).
FIG. 22 indicates graphs showing the numbers of CD4 and CD8 CAR T cells transduced with mCD19BBz (CD19) or mCD19BBz-CTC28 (CD19-CTC28) in the A20 cell tumor mouse model.
FIG. 23 illustrates graphs showing the amounts of IFN-γ, IL-2, and TNF-α secreted by CD4 and CD8 CAR T cells transduced with mCD19BBz (CD19) or mCD19BBz-CTC28 (CD19-CTC28) in the A20 cell tumor mouse model.
FIG. 24 illustrates graphs quantifying the amounts of IFN-γ, IL-2, and TNF-α secreted by CD4 and CD8 CAR T cells transduced with mCD19BBz (CD19) or mCD19BBz-CTC28 (CD19-CTC28) in the A20 cell tumor mouse model.
FIG. 25 illustrates a graph showing the changes in tumor size in mice co-administered with a 1:1 mixture of CD4 or CD8 mCD19BBz CAR T cells with (+) or without (-) CTC28 in the A20 cell tumor mouse model.
FIG. 26 indicates graphs showing the numbers of CAR T cells in mice co-administered with a 1:1 mixture of CD4 or CD8 mCD19BBz CAR T cells with (+) or without (-) CTC28 in the A20 cell tumor mouse model.
FIG. 27 illustrates graphs showing the RNA-seq analysis of CD4 and CD8 CAR T cells transduced with mCD19BBz (CD19) or mCD19BBz-CTC28 (CD19-CTC28) in the A20 cell tumor mouse model.
FIG. 28 illustrates a graph showing the changes in tumor size in mice treated with CAR T cells transduced with mCD19BBz (CD19) or mCD19BBz-CTC28 (CD19-CTC28) together with an IL-2 neutralizing antibody (α-IL2) in the A20 cell tumor mouse model.
FIG. 29 indicates graphs showing the numbers of CAR T cells in mice treated with CAR T cells transduced with mCD19BBz (CD19) or mCD19BBz-CTC28 (CD19-CTC28) together with an IL-2 neutralizing antibody (α-IL2) in the A20 cell tumor mouse model.
FIG. 30 indicates graphs showing the numbers of CD4 and CD8 CAR T cells in the peripheral blood of mice after administration of CAR T cells transduced with mCD19BBz (CD19) or mCD19BBz-CTC28 (CD19-CTC28) in the mouse model.
FIG. 31 illustrates graphs showing the changes in body weight of mice after administration of CAR T cells transduced with mCD19BBz (CD19) or mCD19BBz-CTC28 (CD19-CTC28) in the mouse model.
FIG. 32 illustrates graphs showing the numbers of CAR T cells and B cells in the blood of mice after administration of CAR T cells transduced with mCD19BBz (CD19) or mCD19BBz-CTC28 (CD19-CTC28) and treatment with an anti-CTLA4 antibody in the mouse model.
FIG. 33 shows the gene construct structures of the human 41BB-based CAR (hCD19BBz) and the human CTC28 co-expressed with the CAR (hCD19BBz-CTC28).
FIG. 34 shows graphs analyzing the expression of CAR and CTC28 proteins on the surface of T cells transduced with the human CAR (hCD19BBz) or the human CTC28 (CD19-CTC28 CAR).
FIG. 35 shows graphs analyzing the tumor killing ability of T cells transduced with the human hCD19BBz (CD19) or hCD19BBz-CTC28 (CD19-CTC28) against Raji cells.
FIG. 36 illustrates graphs showing the amounts of IFN-γ, IL-2, and TNF-α secreted by T cells transduced with the human hCD19BBz (CD19) or hCD19BBz-CTC28 (CD19-CTC28) when co-cultured with Raji cells.
FIG. 37 illustrates graphs showing the changes in tumor size in mice injected with T cells transduced with the human hCD19BBz (CD19) or hCD19BBz-CTC28 (CD19-CTC28).
FIG. 38 illustrates graphs showing the numbers of CD4 and CD8 CAR T cells in NSG immunodeficient mice injected with the human hCD19BBz (CD19) or hCD19BBz-CTC28 (CD19-CTC28).

### [Best Mode for Carrying Out the Invention]

One aspect provides a construct comprising:
(i) a first gene encoding a fusion protein (CTLA4-CD28 fusion protein) comprising a CTLA4 (Cytotoxic T Lymphocyte Antigen-4) protein or a domain thereof; and CD28 or a domain thereof; and
(ii) a second gene encoding a chimeric antigen receptor (CAR) comprising an antigen binding domain; an extracellular spacer domain; a transmembrane domain; and an intracellular signaling domain.

As used herein, the term "construct" refers to a macromolecule or complex of molecules comprising a polynucleotide to be delivered to a host cell, either *in vitro, in vivo,* or *ex vivo.* The term "vector" as used herein refers to any nucleic acid construct capable of directing the delivery or transfer of a foreign genetic material to target cells, where said polynucleotide can be replicated and/or expressed. The term "vector" as used herein comprises the construct to be delivered. A vector can be a linear or a circular molecule. A vector can be integrating or non-integrating. The major types of vectors include, but are not limited to, plasmids, episomal vector, viral vectors, non-viral vectors, cosmids, and artificial chromosomes. Viral vectors include, but are not limited to, adenovirus vector, adeno-associated virus vector, retrovirus vector, lentivirus vector, Sendai virus vector, and the like.

As used herein, the term "encoding" refers to the inherent property of specific sequences of nucleotides in a polynucleotide, such as a gene, a cDNA, or a mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as encoding the protein or other product of that gene or cDNA.

As used herein, the term "extracellular domain" refers to a domain that protrudes outside the cell and binds to a ligand or the like.

As used herein, the term "transmembrane domain" refers to a domain that connects an extracellular domain to an intracellular domain and is located in the cell membrane.

As used herein, the term "intracellular domain" refers to a domain that is located inside the cell membrane of a cell, i.e., in the cytoplasm, and that binds an extracellular domain and a ligand or the like to transmit a signal into the cell.

One aspect provides a fusion protein in which the intracellular signaling domain of a T-cell inhibitory receptor is removed and the intracellular signaling domain of the T-cell activating surface protein CD28 is fused.

In some embodiments, the fusion protein may be an immune regulatory protein.

In some embodiments, the fusion protein may comprise an extracellular domain, a transmembrane domain, and an intracellular domain.

In some embodiments, the CTLA4-CD28 fusion protein may comprise, but is not limited to, a CTLA4 extracellular domain and a CD28 intracellular domain.

In some embodiments, the CTLA4-CD28 fusion protein may be fused by the transmembrane domain of CTLA4 or CD28, but is not limited thereto.

In some embodiments, the CTLA4-CD28 fusion protein may comprise, but is not limited to, a CTLA4 extracellular domain-CTLA4 transmembrane domain-CD28 intracellular domain, or a CTLA4 extracellular domain-CD28 transmembrane domain-CD28 intracellular domain.

Specifically, a CTLA molecule comprises the extracellular domain of cytotoxic T-lymphocyte-associated antigen 4. The extracellular domain of CTLA4 comprises a portion of the CTLA protein that recognizes and binds to at least one B7 (CD80/86) antigen, such as the B7 antigen expressed on B cells and antigen-presenting cells (APCs). The extracellular domain may also comprise a fragment or derivative of CTLA4 that bind to the B7 antigen. The CTLA4 extracellular domain can recognize and bind to CD80 (B7-1) and/or CD86 (B7-2). The extracellular domain may comprise a fragment or derivative of CTLA4 that bind to CD80 and/or CD86.

In some embodiments, the fusion protein may comprise, but is not limited to, a CTLA4 extracellular domain-CTLA4 transmembrane domain-CD28 intracellular domain, or a CTLA4 extracellular domain-CD28 transmembrane domain-CD28 intracellular domain.

In some embodiments, the amino acid sequence encoding the CTLA4-CD28 chimeric protein may include the amino acid sequences of SEQ ID NOs: 1 to 7, or a portion thereof, or an amino acid sequence having at least 70%, 75%, 80%, 85%, 90%, 95%, 96%, 97%, 98%, or 99% identity thereto.

**[Table 1]**

| SEQ ID NO: | Type | Sequence |
|---|---|---|
| 1 | CTLA4-human | |
| 2 | CTLA4-mouse | MACLGLRRYK AQLQLPSRTW PFVALLTLLF IPVFSEAIQV TQPSVVLASS HGVASFPCEY SPSHNTDEVR VTVLRQTNDQ MTEVCATTFT EKNTVGFLDY PFCSGTFNES RVNLTIQGLR AVDTGLYLCK VELMYPPPYF VGMGNGTQIY VIDPEPCPDS DFLLWILVAV SLGLFFYSFL VTAVSLSKML KKRSPLTTGV YVKMPPTEPE CEKQFQPYFI PIN |
| 3 | CD28-human | MLRLLLALNL FPSIQVTGNK ILVKQSPMLV AYDNAVNLSC KYSYNLFSRE FRASLHKGLD SAVEVCVVYG NYSQQLQVYS KTGFNCDGKL GNESVTFYLQ NLYVNQTDIY FCKIEVMYPP PYLDNEKSNG TIIHVKGKHL CPSPLFPGPS KPFWVLVVVG GVLACYSLLV TVAFIIFWVR SKRSRLLHSD YMNMTPRRPG PTRKHYQPYA PPRDFAAYRS |
| 4 | CD28-mouse | MTLRLLFLAL NFFSVQVTEN KILVKQSPLL VVDSNEVSLS CRYSYNLLAK EFRASLYKGV NSDVEVCVGN GNFTYQPQFR SNAEFNCDGD FDNETVTFRL WNLHVNHTDI YFCKIEFMYP PPYLDNERSN GTIIHIKEKH LCHTQSSPKL FWALVVVAGV LFCYGLLVTV ALCVIWTNSR RNRLLQSDYM NMTPRRPGLT RKPYQPYAPA RDFAAYRP |
| 5 | CTLA4-CD28 chimera-human | MACLGFQRHK AQLNLATRTW PCTLLFFLLF IPVFCKAMHV AQPAVVLASS RGIASFVCEY ASPGKATEVR VTVLRQADSQ VTEVCAATYM MGNELTFLDD SICTGTSSGN QVNLTIQGLR AMDTGLYICK VELMYPPPYY LGIGNGTQIY VIDPEPCPDS DFLLWILAAV SSGLFFYSFL LTAVSLSKMV RSKRSRLLHS DYMNMTPRRP GPTRKHYQPY APPRDFAAYR S |
| 6 | CTLA-CD28 chimera-mouse | MACLGLRRYK AQLQLPSRTW PFVALLTLLF IPVFSEAIQV TQPSVVLASS HGVASFPCEY SPSHNTDEVR VTVLRQTNDQ MTEVCATTFT EKNTVGFLDY PFCSGTFNES RVNLTIQGLR AVDTGLYLCK VELMYPPPYF VGMGNGTQIY VIDPEPCPDS DFLLWILVAV SLGLFFYSFL VTAVSLSKMT NSRRNRLLQS DYMNMTPRRP GLTRKPYQPY APARDFAAYR P |
| 7 | CTLA4-CD28 chimera-human | MACLGFQRHK AQLNLATRTW PCTLLFFLLF IPVFCKAMHV AQPAVVLASS RGIASFVCEY ASPGKATEVR VTVLRQADSQ VTEVCAATYM MGNELTFLDD SICTGTSSGN QVNLTIQGLR AMDTGLYICK VELMYPPPYY LGIGNGTQIY VIDPEPCPDS DFWVLVVVGG VLACYSLLVT VAFIIFWVRS KRSRLLHSDY MNMTPRRPGP TRKHYQPYAP PRDFAAYRS |

In some embodiments, said CTLA4 may comprise an amino acid sequence of human derived SEQ ID NO: 1, an amino acid sequence of mouse derived SEQ ID NO: 2, a portion thereof, or an amino acid sequence having at least 70%, 75%, 80%, 85%, 90%, 95%, 96%, 97%, 98%, or 99% identity thereto. In some embodiments, in the amino acid sequences of SEQ ID NO: 1 and SEQ ID NO: 2, the amino acid sequences from positions 1 to 161 may represent an extracellular domain, i.e., a portion that binds to a ligand such as B7, the amino acid sequences from positions 162 to 189 may represent a transmembrane domain, and the amino acid sequences from positions 190 to 223 may represent an intracellular domain.

In some embodiments, said CD28 may comprise an amino acid sequence of human-derived SEQ ID NO: 3, an amino acid sequence of mouse-derived SEQ ID NO: 4, a portion thereof, or an amino acid sequence having at least 70%, 75%, 80%, 85%, 90%, 95%, 96%, 97%, 98%, or 99% identity thereto.

In some embodiments, in the amino acid sequence of SEQ ID NO: 3, the sequence of amino acids from positions 1 to 152 represents an extracellular domain, i.e., a portion that binds to a ligand such as B7, the sequence of amino acids from positions 153 to 178 represents a transmembrane domain, and the sequence of amino acids from positions 179 to 220 represents an intracellular domain, In the amino acid sequence of SEQ ID NO: 4, the amino acid sequence from positions 1 to 150 may represent an extracellular domain, i.e., a portion that binds to a ligand such as B7, the amino acid sequence from positions 151 to 176 may represent a transmembrane domain, and the amino acid sequence from positions 177 to 218 may represent an intracellular domain.

In some embodiments, the CLTA4-CD28 fusion protein (also referred to herein as "CTC28") can be fused by the transmembrane domain of CTLA4 or CD28. Specifically, when the transmembrane domain of CTLA4 is utilized, it will be apparent to those skilled in the art that it is also within the scope of the present invention if the extracellular domain and transmembrane domain of CTLA4 further comprise some sequences of the intracellular domain of CTLA4, or if the intracellular domain of CD28 further comprise some sequences of the CD28 transmembrane domain, to the extent that the intracellular inhibitory signal of CTLA4 is not transmitted.

In some embodiments, it will also be apparent to those skilled in the art that when the transmembrane domain of CD28 is utilized in the CTLA4-CD28 chimeric protein, it is also within the scope of the present invention if a portion of the extracellular domain of CD28 further comprises the intracellular domain and transmembrane domain of CD28, or if the extracellular domain of CTLA4 further comprises a portion of the sequence of the CTLA4 transmembrane domain, without affecting the binding of CTLA4 to the ligand.

In some embodiments, an example of said CTLA4-CD28 chimeric protein may comprise an amino acid sequence of any one of SEQ ID NO: 5 to SEQ ID NO: 7, a portion thereof, or an amino acid sequence having at least 70%, 75%, 80%, 85%, 90%, 95%, 96%, 97%, 98%, or 99% identity thereto.

In some embodiments, the CTLA4 extracellular domain-CTLA4 transmembrane domain-CD28 intracellular domain can be, but is not limited to, the amino acid sequence of SEQ ID NO: 5, the amino acid sequence of SEQ ID NO: 6, a portion thereof, or an amino acid sequence having at least 70%, 75%, 80%, 85%, 90%, 95%, 96%, 97%, 98%, or 99% identity thereto.

In some embodiments, the CTLA4 extracellular domain-CD28 transmembrane domain-CD28 intracellular domain may be, but is not limited to, the amino acid sequence of SEQ ID NO: 7, a portion thereof, or an amino acid sequence having at least 70%, 75%, 80%, 85%, 90%, 95%, 96%, 97%, 98%, or 99% identity thereto.

In some embodiments, said CTLA4-CD28 chimeric protein may comprise a fusion protein disclosed in Korean Patent Laid-open Publication No. 10-2013-0045824. Specifically, the CTLA4-CD28 chimeric protein may comprise the amino acid sequence of SEQ ID NOS: 1 to 7 of the above publication, a portion thereof, or an amino acid sequence having at least 70%, 75%, 80%, 85%, 90%, 95%, 96%, 97%, 98%, or 99% identity thereto.

In some embodiments, the CLTA4-CD28 chimeric protein may be a fusion protein in which the intracellular inhibitory signaling domain of CTLA4 has been removed and the intracellular activating signaling domain of a CD28 protein has been fused.

When a ligand is bound to CTLA4 of the fusion protein, the intracellular activation signal transduction domain of CD28 contained in the CTLA4-CD28 chimeric protein may change the inhibitory signal of T cell activity caused by the binding of CTLA4 to the ligand to a stimulatory signal.

Therefore, the CLTA4-CD28 chimeric protein can not only overcome T cell tolerance by cancer cells, but also maximize the activation of T cells to enhance anti-cancer ability.

In some embodiments, the chimeric antigen receptor (also referred to herein as "CAR") is a protein that fuses a membrane or intracellular signal transduction site of a T cell activating protein (CD3-zeta chain, CD28, 41BBL, Ox40, ICOS, high-affinity receptor for IgE (FcεRI), and other T cell activating proteins) with an antigen binding domain.

In some embodiments, the chimeric antigen receptor can be (a) a chimeric antigen receptor (first generation CAR) comprising an antigen binding domain; an extracellular spacer domain; a transmembrane domain; and a CD3zeta intracellular signaling domain; or (b) a chimeric antigen receptor (4-1BB-based second generation CAR) comprising an antigen binding domain; an extracellular spacer domain; a transmembrane domain; a 4-1BB intracellular domain; and a CD3zeta intracellular signaling domain.

Specifically, a first generation CAR may comprise an extracellular domain containing an antigen recognition site specifically expressed by cancer cells, a transmembrane domain, and an intracellular signaling domain, and utilize only CD3ζ as the signal transduction domain.

Specifically, first generation CARs comprise an extracellular domain comprising a region that recognizes an antigen specifically expressed in cancer cells, a transmembrane domain, and an intracellular signaling domain, and may use CD3ζ only as the signaling domain. Second generation CARs comprising a co-stimulatory domain (CD28 or CD137/4-1BB) and CD3ζ, which are coupled to each other, was prepared in order to improve the response to immune cells, and the number of CAR-containing immune cells remaining in the body was significantly increased compared to the first generation CARs. Third generation CARs used two or more co-stimulatory domains. The co-stimulatory domain may be coupled with 4-1BB, CD28, or OX40 in order to achieve expansion and persistence of immune cells comprising CAR *in vivo.* Fourth generation CARs may include an additional gene encoding a cytokine, such as IL-12 or IL-15, to allow further expression of the CAR-based immunoprotein of cytokine. Fifth generation CARs further include an interleukin receptor chain, such as IL-2Rβ, to enhance immune cells.

In some embodiments, the chimeric antigen receptor may have the structure of a first generation CAR to a fifth generation CAR.

In some embodiments, the chimeric antigen receptor may not comprise a CD28 intracellular region.

In some embodiments, the extracellular spacer domain of said chimeric antigen receptor may further comprise a hinge domain.

In some embodiments, the hinge domain may be comprised of any oligopeptide or polypeptide, and may comprise 1 to 100 amino acid residues, and preferably 10 to 70 amino acid residues, but is not limited thereto.

In some embodiments, the extracellular linker of said chimeric antigen receptor may further comprise a hinge domain.

In some embodiments, the chimeric antigen receptor may comprise a gene sequence or amino acid sequence of SEQ ID Nos: 8 to 15; a portion thereof; or a gene sequence or amino acid sequence having at least 70%, 75%, 80%, 85%, 90%, 95%, 96%, 97%, 98%, or 99% identity thereto.

**[Table 2].**

| SEQ ID NO: | Type | Sequence |
|---|---|---|
| 8 | CD19z-mouse | atggccctcac cgttgacccg cttctgtcg ctgaacctgc tgctgctgggg tgagtcgatt atcctgggga gtggggagaagc tgacatccag atgacccaga gccctgccag cctgtctacc agcctgggggg agacagtgac catccagtgt caggccagcg aggacatcta cctctggccctg gcttggtatc agcagaagcc cggcaagagc cctcagctgc tgatctacgg cggccagcgac ctgcaggacg gcgtgcctag cagattcagc ggcagcgggct ccggaaccca gtacagcctg aagatcaccca gcatgcagac cggaggacgag ggcgtgtact tctgccagca aggcctgacc taccctaaa ccttcgggggg aggcaccaag ctggaactga agggcgggggg cggaagtgga gccgggaggat ctgccggcgg aggctctgaa gtgcagctgc agcagtctgg cgctgaaactg gtccgccctg gcactagcgt gaagctgtcc tgcaaggtgt ccgccgacac catcaccttc tactacatgc acttcgtgaa gcagaggcca ggacagggcc tggaatggat cggcagaatc gaccctgagg acgagagcac caagtacagc gagaagttca agaacaaggc caccctgacc gccgacacca gcagcaacac cgcctacctg aagctgtcta gcctgacctc cgaggacacc gccacctact tttgcatcta cgggggctac tacttcgact actggggcca gggggtgatg gtcaccgtgt ccagcactac taccaagcca gtgctgcgaa ctccctcacc tgtgcaccct accgggacat ctcagcccca gagaccagaa gattgtcggc cccgtggctc agtgaagggg accggattgg acttcgcctg tgatatttac atctgggcac ccttggccgg aatctgggtg gcccttctgc tgtccttgat catcactctc atctgcagag caaaattcag caggagtgca gagactgctg ccaacctgca ggaccccaac cagctctaca atgagctcaa tctagggcga agagaggaat atgacgtctt ggagaagaag cgggctcggg atccagagat gggaggcaaa cagcagagga ggaggaaccc ccaggaaggc gtatacaatg cactgcagaa agacaagatg gcagaagcct acagtgagat cgccacaaaa gcggagaggc ggagaggcaa ggggcacgat ggcctttacc agggtctcag cactgccacc aaggacacct atgatgccct gcatatgcag accctggccc ctcgctaa |
| 9 | | maspltrfls lnllllgesi ilgsgeadiq mtqspaslst slgetvtiqc qasediysgl awyqqkpgks pqlliygasd lqdgvpsrfs gsgsgtqysl kitsmqtede gvyfcqqglt yprtfgggtk lelkgggggsg ggggsgggggse vqlqqsgael vrpgtsvkls ckvsgdtitf yymhfvkqrp gqglewigri dpedestkeys ekfknkatlt adtssntayl klssltsedt atyfciyggy yfdywgqgvm vtvsstttkp vlrtpspvhp tgtsqpqrpe dcrprgsvkg tgldfacdy iwaplagicv alllsliitl icrakfsrsa etaanlqdpn qlynelnlgr reeydvlekk rardpemggk qqrrrnpqeg vynalqkdkm aeayseigtk gerrrgkghd glyqglstat kdtydalhmq tlapr |
| 10 | CD1928z-mouse | atgggggtcc ctacccagct cctgggactg ctcctgctgt ggatcaccga cgccatctgc gacatccaga tgacccagag ccctgccagc ctgtctacca gcctgggcga gacagtgacc atccagtgtc aggccagcga ggacatctac tctggcctgg cttggtatca gcagaagccc ggcaagagcc ctcagctgct gatctacgcc gccagcgacc tgcaggacgg cgtgcctagc agattcagcg gcagcggctc cggaacccag tacagcctga agatcaccag catgcagacc gaggacgagg gcgtgtactt ctgccagcaa ggcctgacct accctagaac cttcggagga gccaccaagc tggaactgaa gggggggaggc ggaagtggag gggggaggatc tggcgggggga GGCTCTGAAG TGCAGCTGCA GCAGTCTGGC GCTGAACTGG TCCGGCCTGG CACTAGCGTG aagctgtcct gcaaggtgtc cggcgacacc atcaccttct actacatgca cttcgtgaag cagaggccag gacagggcct ggaatggatc ggcagaatcg accctgagga cgagagcacc aagtacagcg agaagttcaa gaacaaggcc accctgaccg ccgacaccag cagcaacacc gcctacctga agctgtctag cctgacctcc gaggacaccg ccacctactt ttgcatctac gccgggctact acttcgacta ctggggccag ggcgtgatgg tcaccgtgtc cagcatcgag ttcatgtacc cccctcccta cctggacaac gagagaagca acggcaccat catccacatc aaagaaaagc acctgtgcca cacccagagc agccccaagc tgttctgggc cctggtggtg gtgggccgcg tgctgttctg ttacgccctg ctggtcacag tggccctgtg cgtgatctgg accaacagca gaagaaacag aggcgggcag agcgactaca tgaacatgac ccccagaagg ccaggcctga ccagaaagcc ctaccagccc tacgcccctg ccagagactt cgccgcctac agacccagag ccaagttcag cagatccgcc gagacagccg ccaacctgca ggatcccaac cagctgttca acgagctgaa cctgggcaga cgggaggaat tcgacgtgct ggaaaagaag agagccaggg accccgagat ggggggcaag cagcagagaa gaagaaaccc tcaggaaggc gtctacaacg ccctgcagaa agacaagatg gccgaggcct acagcgagat cggcaccaag gccgagagaa gaaggggcaa gggccacgat gccctgttcc agggcctgtc caccgccacc aaggacacct tcgacgccct gcacatgcag accctggccc **ccagatga** |
| 11 | | mgvptqllgl lllwitdaic diqmtqspas lstslgetvt iqcqasediy sglawyqqkp gkspqlliyg asdlqdgvps rfsgsgsgtstq yslkitssmqt edegvyfcqq gltyprtfggg gtklelkggg gsgggggggsggg gsevqlqqsg aelvrpgtsv klsckvsgdt itfyymhfvk qrpgqglewi gridpedest kysekfknka tltadtssnt aylklsslts edtatyfciy ggyyfdywgq gvmvtvssie fmypppyldn ersngtiihi kekhlchtqs spklfwalvv vagvlfcygl lvtvalcviw tnsrrnrggq sdymnmptprr pgltrkpyqp yapardfaay rprakfsrsa etaanlqdpn qlfnelnlgr reefdvlekk rardpemggk qqrrrnnpqeg vynalqkdkm aeayseigtk gerrrgkghd glfqglstat kdtfdalhmq tlapr |
| 12 | CD19BBz-mouse | atggccctcac cgttgacccg cttctgtcg ctgaacctgc tgctgctgggg tgagtcgatt atcctgggga gtggggagaagc tgacatccag atgacccaga gccctgccag cctgtctacc agcctgggggg agacagtgac catccagtgt caggccagcg aggacatcta cctctggccctg gcttggtatc agcagaagcc cggcaagagc cctcagctgc tgatctacgg cggccagcgac ctgcaggacg gcgtgcctag cagattcagc ggcagcgggct ccggaaccca gtacagcctg aagatcacca gcatgcagac cggaggacgag ggcgtgtact tctgccagca aggcctgacc taccctaaa ccttcgggggg aggcaccaag ctggaactga agggcggggagg cggaagtgga gccgggaggat ctgccggcgg aggctctgaa gtgcagctgc agcagtctgg cgctgaaactg gtccgccctg gcactagcgt gaagctgtcc tgcaaggtgt ccgccgacac catcaccttc tactacatgc acttcgtgaa gcagaggcca ggacagggcc tggaatggat cggcagaatc gaccctgagg acgagagcac caagtacagc gagaagttca agaacaaggc caccctgacc gccgacacca gcagcaacac cgcctacctg aagctgtcta gcctgacctc cggaggacacc gccacctact tttgcatcta cgggggggctac tacttcgact actgggggggcca gggggtgatg gtcaccgtgt ccagcactac taccaagcca gtgctgcgaa ctccctcacc tgtgcaccct accggggacat ctcagcccca gagacccagaa gattgtcgggc cccgtgggctc agtgaaggggg accggattgg acttcgccctg tgatatttac atctggggccac ccttgggccgg aatctgggtgg gcccttctgc tgtccttgat catcactctc atctgcaaac ggggcagaaa gaaaactcctg tatatattca aaaacccatt tatgagacca gtacaaacta ctcaagagga agatggctgt agctgccgat ttccagaaga agaagaagga ggatgtgaac tgagagcaaa attcagcagg agtgcagaga ctgctgccaa cctgcaggac cccaacccagc tctacaatga gctcaatcta GGGCGAAGAG AGGAATATGA CGTCTTGGAG AAGAAGCGGG CTCGGGGATCC AGAGATGGGA gccaaacagc agaggaggag gaacccccag gaaggggtat acaatgcact gcagaaagac aagatggcag aagcctacag tgagatcggc acaaaaggcg agaggcggggag aggcaagggg cacgatggggcc tttaccaggg tctcagcact gccaccagg acacctatga tgccctgcat atgcagaccc tggcccctcg ctaa |
| 13 | | maspltrfls lnllllgesi ilgsgeadiq mtqspaslst slgetvtiqc qasediysgl awyqqkpgks pqlliygasd lqdgvpsrfs gsgsgtqysl kitsmqtede gvyfcqqglt yprtfgggtk lelkgggggsg ggggsgggggse vqlqqsgael vrpgtsvkls ckvsgdtitf yymhfvkqrp gqglewigri dpedestkeys ekfknkatlt adtssntayl klssltsedt atyfciyggy yfdywgqgvm vtvsstttkp vlrtpspvhp tgtsqpqrpe dcrprgsvkg tgldfacdy iwaplagicv alllsliitl ickrgrkkkll yifkqpfmrp vqttqeedgc scrfpeeeeg gcelrakfsr saetaanlqd pnqlynnelnl grreeydvle kkrardpemg gkqqrrrnpq egvynalqkd kmaeayseig tkgerrrgkg hdglyqglst atkdtydalh MQTLAPR |
| 14 | CD19BBz-human | atgcccttac cagtgaccgc cttgctcctg ccgctggcct tgctgctcca cggccgccagg ccggacatcc agatgacaca gactacatcc tccctgtctg cctctctgggg agacagagtc accacccagtt gcaggggcaag tcaggacatt agtaaatatt taattggta tcagcagaaa ccagatggaa cgttaaaact cctgatctac catacatcaa gattacactc aggagtccca tcaaggttca gtggcagtgg gtctggaaca gattattctctc tcaccattag caacctggag caagaagata ttgccactta cttttgccaa caggggtaata cgcttccgta cacgttcgga gggggggacca agctggggagat cacaggtgggc ggtgggctcgg gcgggggggggg gtcgggggggc ggcgggatctg aggtgaaact gcaggagtca ggacctggcc tggtgggcgcc ctcacagagc ctgtccgtca catgcactgt ctcagggggtc tcattacccg actatggtgt aagctggatt cgccagcctc cacgaaagggg tctggggagtgg ctggggagtaa tatgggggtag tgaaaccaca tactataatt cagctctcaa atccagactg accatcatca aggacaactc caagagccaa gtttttcttaa aaatgaacag tctgcaaact gatgacacag ccatttaacta ctgtgccaaa catchattact acggtggggtag cattgctatg gactactgggg gccaaggaac ctcagtcacc gtctcctcaa ccacgacgcc agcgccgcga ccaccaacac cggcgcccac catcgcgtcg cagcccctgt ccctgcgccc agaggggtgc cggccagcgg cgggggggccgc agtgcacacg agggggggggctgg acttcgccctg tgatatctac atctgggggccgc ccttgggccgg gacttgtggg gtccttctcc tgtcactggt tatcaccctt tactgcaaac ggggcagaaa gaaaactcctg tatatattca aaaacccatt tatgagacca gtacaaacta ctcaagagga agatgggctgt agctgccgat ttccagaaga agaagaagaaga ggatgtgaac tgagagtgaa gtttcagcagg agcgcagacg cccccgcgta caagcaggggc cagaacccagc tctataacga gctcaatcta ggacgaagag aggagtacga tgttttggac aagagacgtg gccgggggaccc tgagatggggg gaaaagccga gaaggaagaa cccctcaggaa ggcctgtaca atgaactgca gaaagataag atggcggggggg cctacagtga gattgggggatg aaaggcggagc gccgggagggg caaggggggccac gatggccttt accagggtct cagtacagcc accaaggaca cctacgacgc ccttcacatg CAGGCCCT |
| 15 | | malpvtalll plalllhaar pdiqmtqtts slsaslgdrv tiscrasqdi skylnwyqqk pdgtvkllyy htsrlhsgvp srfsgsgsgt dysltisnle qediatyfcq qgntlpytfg ggtkleitgg ggsggggsgg ggsevklqes gpglvapsqs lsvtctvsgv slpdygvswi rqpprkglew lgviwgsett yynsalksrl tiikdnsksq vflkmnslqt ddtaiyycak hyyyggsyam dywgqgtsvt vssttptapr pptpaptias qplslrpeac rpaaggavht rgldfacdy iwaplagtcg vlllslvitl yckrgrkkll yifkqpfmrp vqttqeedgc scrfpeeeeg gcelrvkfsr sadapaykqg qnqlynelnl grreeydvld krrgrdpemg gkprrknpqe glynelqkdk maeayseigm kgerrrgkgh dglyqglsta tkdtydalhm QALPPR |

In some embodiments, the intracellular signaling domain of said chimeric antigen receptor may refer to a portion located inside the cell membrane of an immune cell, i.e., in the cytoplasm, where the antigen binding domain contained in the extracellular domain, upon binding to a target antigen, transmits an intracellular signal to activate an immune response of the immune cell. In some embodiments, said intracellular signaling domain is selected from CD3 zeta (ζ), CD3 gamma (γ), CD3 delta (δ), CD3 epsilon (ε), FcR gamma, FcR beta, CD5, CD22, CD79a, CD79b, and CD66d, but is not limited thereto. In some embodiments, the intracellular signaling domain of said chimeric antigen receptor is CD3 zeta (ζ).

In some embodiments, said intracellular signaling domain may be characterized by further comprising, but not limited to, a co-stimulatory domain. A co-stimulatory domain according to the present invention may comprise one or more co-stimulatory domains selected from the group consisting of ligands that specifically bind to CD2, CD7, CD27, CD28, CD30, CD40, 4-1BB (CD137), OX40 (CD134), ICOS, LFA-1, GITR, MyD88, DAP1, PD-1, LIGHT, NKG2C, B7-H3, and CD83, but is not limited thereto.

In some embodiments, said chimeric antigen receptor may be characterized by comprising one or more intracellular signaling domains and one or more co-stimulatory domains.

In some embodiments, said co-stimulatory domain may be 4-1BB.

In some embodiments, the chimeric antigen receptor may comprise the amino acid sequence of SEQ ID NO: 9, SEQ ID NO: 13, or SEQ ID NO: 15; a portion thereof; or an amino acid sequence having at least 70%, 75%, 80%, 85%, 90%, 95%, 96%, 97%, 98%, or 99% identity thereto.

In some embodiments, the antigen binding domain can be, but is not limited to, an antibody, an antigen-binding fragment thereof, a ligand protein that binds to an antigen, or a domain thereof.

In some embodiments, the antigen binding domain may comprise an antibody or an antigen-binding fragment thereof that specifically binds to one or more antigens selected from the group consisting of, but not limited to

4-1BB, BCMA, BAFF, B7-H3, B7-H6, CA9, CTAG1B, CEA, cyclin, cyclin A2, cyclin B1, CCL-1, CCR4, CD3, CD4, CD19, CD20, CD22, CD23, CD24, CD30, CD33, CD38, CD40, CD44, CD44v6, CD44v7/8, CD52, CD58, CD62, CD79A, CD79B, CD80, CD123, CD133, CD138, CD171, CSPG4, CLDN18, CLDN18.2, CLDN6, CTLA-4, c-Met, DLL3, EGFR, tEGFR, EGFRvIII, EPG-2, EPG-40, ephrin B2, EPHA2, estrogen receptor, Fc receptor, FCRL5, FGF23, FBP, FOLR1, FOLR2, GD2, ganglioside GD3, gp100, GPC3, GPCR5D, GM-CSF, Her2/neu, Her3, Her4, erbB dimers, HMW-MAA, HBsAg, HLA-A1, HLA-A2, IL-22Ra, IL-13Ra2, ICOS, IGF-1 receptor, integrin αvβ6, interferon receptor, IFNγ, IL-2R, IL-4R, IL-5R, IL-6R, IL-17RA, IL-31R, IL-36R, kdr, L1-CAM, CE7 epitope of L1-CAM, LRRC8A, Lewis Y, LAG3, MAGEA1, MAGEA3, MAGEA6, MAGEA10, MSLN, CMV, MUC1, NKG2D ligand, MART-1, NGF, NCAM, NRP-1, NRP-2, carcinoembryonic antigen, PD-L1, PRAME, progesterone receptor, prostate-specific antigen, PSCA, PSMA, RANKL, ROR1, SLAMF7, survivin, TPBG, TAG72, TRP1, TRP2, and Wilms tumor 1 (WT1).

In some embodiments, an antigen-binding fragment refers to a fragment that retains antigen-binding function and may be a single-chain variable fragment (scFv), (scFv)₂, Fv, Fab, Fab', F(ab')₂, nanobody, or combinations thereof, but is not limited thereto.

A "single-chain Fv" or "scFv" antibody fragment comprises the VH and VL domains of an antibody, which are present within a single polypeptide chain. The Fv polypeptide may additionally contain a polypeptide linker between the VH and VL domains that allows the scFv to form the targeted structure for antigen binding.

An "Fv" fragment is an antibody fragment containing a complete antibody recognition and binding site. This region consists of one heavy chain variable domain and one light chain variable domain tightly and virtually covalently associated as a dimer, e.g., scFv.

A "Fab" fragment contains variable and constant domains of the light chain and variable and first constant domain (CH1) of the heavy chain. "F(ab')₂" antibody fragments generally comprise a pair of Fab fragments covalently linked near their carboxy terminus by a hinge cysteine between them.

A "nanobody" is a fragment containing a monomeric variable antibody domain. It consists of low molecular weight fragments mainly derived from antibody domains of camels and similar animals that show target specificity with only monomeric heavy chains.

In some embodiments, the chimeric antigen receptor may further comprise a signal peptide at the N-terminus of the antigen binding domain, but is not limited to. In the present invention, the signal peptide may be derived from molecules selected from the group consisting of CD8α, GM-CSF receptor α, Ig-kappa, and IgG1 heavy chain, but is not limited thereto. Preferably, it may be a CD8α signal peptide, and the CD8α signal peptide may comprise an amino acid sequence represented by SEQ **ID** NO: 25, a portion thereof, or an amino acid sequence having at least 70%, 75%, 80%, 85%, 90%, 95%, 96%, 97%, 98%, or 99% identity thereto.

In some embodiments, the construct may further comprise a promoter sequence, wherein the first gene and the second gene may be operably linked to the promoter sequence, but is not limited thereto.

In some embodiments, the first gene encoding the CTLA4-CD28 fusion protein and the second gene encoding the chimeric antigen receptor may be linked to each other by a 2A peptide sequence or an IRES sequence, or the first and second genes may each be operably linked to independent promoters, but is not limited thereto.

As used herein, the term "operably linked" means that the first gene and second gene are functionally connected to their respective promoters or to one promoter such that nucleic acid sequences having promoter activity initiate and mediate transcription of the first gene or second gene. Operable linkage can be produced using genetic recombination techniques known in the art.

Another aspect provides a vector comprising said construct.

In some embodiments, the vector may be a viral vector or a non-viral vector, but is not limited thereto.

In some embodiments, the viral vector may be any one selected from the group consisting of retrovirus, lentivirus, adenovirus, adeno-associated virus, and vaccinia virus, but not limited to.

The viral or non-viral vector can be used without limitation as long as it can transduce or transfect animal cells, particularly T-cells, by infection.

The virus may be capable of carrying two or more genes. Specifically, the virus may be a retrovirus, a lentivirus, an adenovirus, or an adeno-associated virus. In the working examples, a retrovirus and a lentivirus were used, but are not limited thereto. Specifically, two gene groups including a gene encoding a CLTA4-CD28 chimeric protein and a gene encoding a chimeric antigen receptor were carried in a retrovirus and a lentivirus.

Preferred non-viral vectors include, but are not limited to, those utilizing a transposon system (Hackett et al., US 6,489,458 B), and it will be apparent to a person of ordinary skill in the art that any of the non-viral vectors conventionally available may be used for the purposes of the present invention.

In some embodiments, said viral or non-viral vector may comprise genes for a CTLA4-CD28 chimeric protein and a chimeric antigen receptor. Specifically, the vector may comprise, but is not limited to, the gene sequence of SEQ ID NO: 16, SEQ ID NO: 20, or SEQ ID NO: 22; a portion thereof; or a gene sequence having at least 70%, 75%, 80%, 85%, 90%, 95%, 96%, 97%, 98%, or 99% identity thereto.

In some embodiments, the construct of the invention may comprise, but is not limited to, the amino acid sequence of SEQ ID NO: 17, SEQ ID NO: 21, or SEQ ID NO: 23; a portion thereof; or an amino acid sequence having at least 70%, 75%, 80%, 85%, 90%, 95%, 96%, 97%, 98%, or 99% identity thereto.

**[Table 3]**

| SEQ ID NO: | Type | Sequence |
|---|---|---|
| 16 | CD19z-CTC28 mouse | ATGGCCTCAC CGTTGACCCG CTTTCTGTCG CTGAACCTGC TGCTGCTGGG TGAGTCGATT ATCCTGGGGA GTGGAGAAGC TGACATCCAG ATGACCCAGA GCCCTGCCAG CCTGTCTACC AGCCTGGGCG AGACAGTGAC CATCCAGTGT CAGGCCAGCG AGGACATCTA CTCTGGCCTG GCTTGGTATC AGCAGAAGCC CGGCAAGAGC CCTCAGCTGC TGATCTACGG CGCCAGCGAC CTGCAGGACG GCGTGCCTAG CAGATTCAGC GGCAGCGGCT CCGGAACCCA GTACAGCCTG |
| | | AAGATCACCA GCATGCAGAC CGAGGACGAG GGCGTGTACT TCTGCCAGCA AGGCCTGACC TACCCTAGAA CCTTCGGAGG AGGCACCAAG CTGGAACTGA AGGGCGGAGG CGGAAGTGGA GGCGGAGGAT CTGGCGGCGG AGGCTCTGAA GTGCAGCTGC AGCAGTCTGG CGCTGAACTG GTCCGGCCTG GCACTAGCGT GAAGCTGTCC TGCAAGGTGT CCGGCGACAC CATCACCTTC TACTACATGC ACTTCGTGAA GCAGAGGCCA GGACAGGGCC TGGAATGGAT CGGCAGAATC GACCCTGAGG ACGAGAGCAC CAAGTACAGC GAGAAGTTCA AGAACAAGGC CACCCTGACC GCCGACACCA GCAGCAACAC CGCCTACCTG AAGCTGTCTA GCCTGACCTC CGAGGACACC GCCACCTACT TTTGCATCTA CGGCGGCTAC TACTTCGACT ACTGGGGCCA GGGCGTGATG GTCACCGTGT CCAGCACTAC TACCAAGCCA GTGCTGCGAA CTCCCTCACC TGTGCACCCT ACCGGGACAT CTCAGCCCCA GAGACCAGAA GATTGTCGGC CCCGTGGCTC AGTGAAGGGG ACCGGATTGG ACTTCGCCTG TGATATTTAC ATCTGGGCAC CCTTGGCCGG AATCTGCGTG GCCCTTCTGC TGTCCTTGAT CATCACTCTC ATCTGCAGAG CAAAATTCAG CAGGAGTGCA GAGACTGCTG CCAACCTGCA GGACCCCAAC CAGCTCTACA ATGAGCTCAA TCTAGGGCGA AGAGAGGAAT ATGACGTCTT GGAGAAGAAG CGGGCTCGGG ATCCAGAGAT GGGAGGCAAA CAGCAGAGGA GGAGGAACCC CCAGGAAGGC GTATACAATG CACTGCAGAA AGACAAGATG GCAGAAGCCT ACAGTGAGAT CGGCACAAAA GGCGAGAGGC GGAGAGGCAA GGGGCACGAT GGCCTTTACC AGGGTCTCAG CACTGCCACC AAGGACACCT ATGATGCCCT GCATATGCAG ACCCTGGCCC CTCGCGGAAG CGGAGCTACT AACTTCAGCC TGCTGAAGCA GGCTGGAGAC GTGGAGGAGA ACCCTGGACC TATGGCTTGT CTTGGACTCC GGAGGTACAA AGCTCAACTG CAGCTGCCTT CTAGGACTTG GCCTTTTGTA GCCCTGCTCA |
| | | CTCTTCTTTT CATCCCAGTC TTCTCTGAAG CCATACAGGT GACCCAACCT TCAGTGGTGT TGGCTAGCAG CCATGGTGTC GCCAGCTTTC CATGTGAATA TTCACCGTCA CACAACACTG ATGAGGTCCG GGTGACTGTG CTGCGGCAGA CAAATGACCA AATGACTGAG GTCTGTGCCA CGACATTCAC AGAGAAGAAT ACAGTGGGCT TCCTAGATTA CCCCTTCTGC AGTGGTACCT TTAATGAAAG CAGAGTGAAC CTCACCATCC AAGGACTGAG AGCTGTTGAC ACGGGACTGT ACCTCTGCAA GGTGGAACTC ATGTACCCAC CGCCATACTT TGTGGGCATG GGCAACGGGA CGCAGATTTA TGTCATTGAT CCAGAACCAT GCCCGGATTC TGACTTCCTC CTTTGGATCC TTGTCGCAGT TAGCTTGGGG TTGTTTTTTT ACAGTTTCCT GGTCACTGCT GTTTCTTTGA GCAAGATGAC AAATAGTAGA AGGAACAGAC TCCTTCAAAG TGACTACATG AACATGACTC CCCGGAGGCC TGGGCTCACT CGAAAGCCTT ACCAGCCCTA CGCCCCTGCC AGAGACTTTG CAGCGTACCG CCCCTGA |
| 17 | | MASPLTRFLS LNLLLLGESI ILGSGEADIQ MTQSPASLST SLGETVTIQC QASEDIYSGL AWYQQKPGKS PQLLIYGASD LQDGVPSRFS GSGSGTQYSL KITSMQTEDE GVYFCQQGLT YPRTFGGGTK LELKGGGGSG GGGSGGGGSE VQLQQSGAEL VRPGTSVKLS CKVSGDTITF YYMHFVKQRP GQGLEWIGRI DPEDESTKYS EKFKNKATLT ADTSSNTAYL KLSSLTSEDT ATYFCIYGGY YFDYWGQGVM VTVSSTTTKP VLRTPSPVHP TGTSQPQRPE DCRPRGSVKG TGLDFACDIY IWAPLAGICV ALLLSLIITL ICRAKFSRSA ETAANLQDPN QLYNELNLGR REEYDVLEKK RARDPEMGGK QQRRRNPQEG VYNALQKDKM AEAYSEIGTK GERRRGKGHD GLYQGLSTAT KDTYDALHMQ TLAPRGSGAT NFSLLKQAGD VEENPGPMAC LGLRRYKAQL QLPSRTWPFV ALLTLLFIPV FSEAIQVTQP SVVLASSHGV ASFPCEYSPS HNTDEVRVTV |
| | | LRQTNDQMTE VCATTFTEKN TVGFLDYPFC SGTFNESRVN LTIQGLRAVD TGLYLCKVEL MYPPPYFVGM GNGTQIYVID PEPCPDSDFL LWILVAVSLG LFFYSFLVTA VSLSKMTNSR RNRLLQSDYM NMTPRRPGLT RKPYQPYAPA RDFAAYRP |
| 18 | CD1928z-CTC28 mouse | ATGGGTGTCC CTACCCAGCT CCTGGGACTG CTCCTGCTGT GGATCACCGA CGCCATCTGC GACATCCAGA TGACCCAGAG CCCTGCCAGC CTGTCTACCA GCCTGGGCGA GACAGTGACC ATCCAGTGTC AGGCCAGCGA GGACATCTAC TCTGGCCTGG CTTGGTATCA GCAGAAGCCC GGCAAGAGCC CTCAGCTGCT GATCTACGGC GCCAGCGACC TGCAGGACGG CGTGCCTAGC AGATTCAGCG GCAGCGGCTC CGGAACCCAG TACAGCCTGA AGATCACCAG CATGCAGACC GAGGACGAGG GCGTGTACTT CTGCCAGCAA GGCCTGACCT ACCCTAGAAC CTTCGGAGGA GGCACCAAGC TGGAACTGAA GGGCGGAGGC GGAAGTGGAG GCGGAGGATC TGGCGGCGGA GGCTCTGAAG TGCAGCTGCA GCAGTCTGGC GCTGAACTGG TCCGGCCTGG CACTAGCGTG AAGCTGTCCT GCAAGGTGTC CGGCGACACC ATCACCTTCT ACTACATGCA CTTCGTGAAG CAGAGGCCAG GACAGGGCCT GGAATGGATC GGCAGAATCG ACCCTGAGGA CGAGAGCACC AAGTACAGCG AGAAGTTCAA GAACAAGGCC ACCCTGACCG CCGACACCAG CAGCAACACC GCCTACCTGA AGCTGTCTAG CCTGACCTCC GAGGACACCG CCACCTACTT TTGCATCTAC GGCGGCTACT ACTTCGACTA CTGGGGCCAG GGCGTGATGG TCACCGTGTC CAGCATCGAG TTCATGTACC CCCCTCCCTA CCTGGACAAC GAGAGAAGCA ACGGCACCAT CATCCACATC AAAGAAAAGC ACCTGTGCCA CACCCAGAGC AGCCCCAAGC TGTTCTGGGC CCTGGTGGTG GTGGCCGGCG TGCTGTTCTG TTACGGCCTG CTGGTCACAG TGGCCCTGTG CGTGATCTGG ACCAACAGCA GAAGAAACAG AGGCGGCCAG AGCGACTACA TGAACATGAC CCCCAGAAGG |
| | | CCAGGCCTGA CCAGAAAGCC CTACCAGCCC TACGCCCCTG CCAGAGACTT CGCCGCCTAC AGACCCAGAG CCAAGTTCAG CAGATCCGCC GAGACAGCCG CCAACCTGCA GGATCCCAAC CAGCTGTTCA ACGAGCTGAA CCTGGGCAGA CGGGAGGAAT TCGACGTGCT GGAAAAGAAG AGAGCCAGGG ACCCCGAGAT GGGCGGCAAG CAGCAGAGAA GAAGAAACCC TCAGGAAGGC GTCTACAACG CCCTGCAGAA AGACAAGATG GCCGAGGCCT ACAGCGAGAT CGGCACCAAG GGCGAGAGAA GAAGGGGCAA GGGCCACGAT GGCCTGTTCC AGGGCCTGTC CACCGCCACC AAGGACACCT TCGACGCCCT GCACATGCAG ACCCTGGCCC CCAGAGGAAG CGGAGCTACT AACTTCAGCC TGCTGAAGCA GGCTGGAGAC GTGGAGGAGA ACCCTGGACC TATGGCTTGT CTTGGACTCC GGAGGTACAA AGCTCAACTG CAGCTGCCTT CTAGGACTTG GCCTTTTGTA GCCCTGCTCA CTCTTCTTTT CATCCCAGTC TTCTCTGAAG CCATACAGGT GACCCAACCT TCAGTGGTGT TGGCTAGCAG CCATGGTGTC GCCAGCTTTC CATGTGAATA TTCACCGTCA CACAACACTG ATGAGGTCCG GGTGACTGTG CTGCGGCAGA CAAATGACCA AATGACTGAG GTCTGTGCCA CGACATTCAC AGAGAAGAAT ACAGTGGGCT TCCTAGATTA CCCCTTCTGC AGTGGTACCT TTAATGAAAG CAGAGTGAAC CTCACCATCC AAGGACTGAG AGCTGTTGAC ACGGGACTGT ACCTCTGCAA GGTGGAACTC ATGTACCCAC CGCCATACTT TGTGGGCATG GGCAACGGGA CGCAGATTTA TGTCATTGAT CCAGAACCAT GCCCGGATTC TGACTTCCTC CTTTGGATCC TTGTCGCAGT TAGCTTGGGG TTGTTTTTTT ACAGTTTCCT GGTCACTGCT GTTTCTTTGA GCAAGATGAC AAATAGTAGA AGGAACAGAC TCCTTCAAAG TGACTACATG AACATGACTC CCCGGAGGCC TGGGCTCACT CGAAAGCCTT ACCAGCCCTA CGCCCCTGCC AGAGACTTTG CAGCGTACCG CCCCTGA |
| 19 | | MGVPTQLLGL LLLWITDAICDIQMTQSPAS LSTSLGETVT IQCQASEDIY SGLAWYQQKP GKSPQLLIYG ASDLQDGVPS RFSGSGSGTQ YSLKITSMQT EDEGVYFCQQ GLTYPRTFGG GTKLELKGGG GSGGGGSGGG GSEVQLQQSG AELVRPGTSV KLSCKVSGDT ITFYYMHFVK QRPGQGLEWI GRIDPEDEST KYSEKFKNKA TLTADTSSNT AYLKLSSLTS EDTATYFCIY GGYYFDYWGQ GVMVTVSSIE FMYPPPYLDN ERSNGTIIHI KEKHLCHTQS SPKLFWALVV VAGVLFCYGL LVTVALCVIW TNSRRNRGGQ SDYMNMTPRR PGLTRKPYQP YAPARDFAAY RPRAKFSRSA ETAANLQDPN QLFNELNLGR REEFDVLEKK RARDPEMGGK QQRRRNPQEG VYNALQKDKM AEAYSEIGTK GERRRGKGHD GLFQGLSTAT KDTFDALHMQ TLAPRGSGAT NFSLLKQAGD VEENPGPMAC LGLRRYKAQL QLPSRTWPFV ALLTLLFIPV FSEAIQVTQP SVVLASSHGV ASFPCEYSPS HNTDEVRVTV LRQTNDQMTE VCATTFTEKN TVGFLDYPFC SGTFNESRVN LTIQGLRAVD TGLYLCKVEL MYPPPYFVGM GNGTQIYVID PEPCPDSDFL LWILVAVSLG LFFYSFLVTA VSLSKMTNSR RNRLLQSDYM NMTPRRPGLT RKPYQPYAPA RDFAAYRP |
| 20 | CD19BBz-CTC28 mouse | ATGGCCTCAC CGTTGACCCG CTTTCTGTCG CTGAACCTGC TGCTGCTGGG TGAGTCGATT ATCCTGGGGA GTGGAGAAGC TGACATCCAG ATGACCCAGA GCCCTGCCAG CCTGTCTACC AGCCTGGGCG AGACAGTGAC CATCCAGTGT CAGGCCAGCG AGGACATCTA CTCTGGCCTG GCTTGGTATC AGCAGAAGCC CGGCAAGAGC CCTCAGCTGC TGATCTACGG CGCCAGCGAC CTGCAGGACG GCGTGCCTAG CAGATTCAGC GGCAGCGGCT CCGGAACCCA GTACAGCCTG AAGATCACCA GCATGCAGAC CGAGGACGAG GGCGTGTACT TCTGCCAGCA AGGCCTGACC TACCCTAGAA CCTTCGGAGG AGGCACCAAG CTGGAACTGA AGGGCGGAGG CGGAAGTGGA |
| | | GGCGGAGGAT CTGGCGGCGG AGGCTCTGAA GTGCAGCTGC AGCAGTCTGG CGCTGAACTG GTCCGGCCTG GCACTAGCGT GAAGCTGTCC TGCAAGGTGT CCGGCGACAC CATCACCTTC TACTACATGC ACTTCGTGAA GCAGAGGCCA GGACAGGGCC TGGAATGGAT CGGCAGAATC GACCCTGAGG ACGAGAGCAC CAAGTACAGC GAGAAGTTCA AGAACAAGGC CACCCTGACC GCCGACACCA GCAGCAACAC CGCCTACCTG AAGCTGTCTA GCCTGACCTC CGAGGACACC GCCACCTACT TTTGCATCTA CGGCGGCTAC TACTTCGACT ACTGGGGCCA GGGCGTGATG GTCACCGTGT CCAGCACTAC TACCAAGCCA GTGCTGCGAA CTCCCTCACC TGTGCACCCT ACCGGGACAT CTCAGCCCCA GAGACCAGAA GATTGTCGGC CCCGTGGCTC AGTGAAGGGG ACCGGATTGG ACTTCGCCTG TGATATTTAC ATCTGGGCAC CCTTGGCCGG AATCTGCGTG GCCCTTCTGC TGTCCTTGAT CATCACTCTC ATCTGCAAAC GGGGCAGAAA GAAACTCCTG TATATATTCA AACAACCATT TATGAGACCA GTACAAACTA CTCAAGAGGA AGATGGCTGT AGCTGCCGAT TTCCAGAAGA AGAAGAAGGA GGATGTGAAC TGAGAGCAAA ATTCAGCAGG AGTGCAGAGA CTGCTGCCAA CCTGCAGGAC CCCAACCAGC TCTACAATGA GCTCAATCTA GGGCGAAGAG AGGAATATGA CGTCTTGGAG AAGAAGCGGG CTCGGGATCC AGAGATGGGA GGCAAACAGC AGAGGAGGAG GAACCCCCAG GAAGGCGTAT ACAATGCACT GCAGAAAGAC AAGATGGCAG AAGCCTACAGTGAGATCGGC ACAAAAGGCG AGAGGCGGAG AGGCAAGGGG CACGATGGCC TTTACCAGGG TCTCAGCACT GCCACCAAGG ACACCTATGA TGCCCTGCAT ATGCAGACCC TGGCCCCTCG CGGAAGCGGA GCTACTAACT TCAGCCTGCT GAAGCAGGCT GGAGACGTGG AGGAGAACCC TGGACCTATG GCTTGTCTTG GACTCCGGAG GTACAAAGCT CAACTGCAGC TGCCTTCTAG GACTTGGCCT TTTGTAGCCC |
| | | TGCTCACTCT TCTTTTCATC CCAGTCTTCT CTGAAGCCAT ACAGGTGACC CAACCTTCAG TGGTGTTGGC TAGCAGCCAT GGTGTCGCCA GCTTTCCATG TGAATATTCA CCGTCACACA ACACTGATGA GGTCCGGGTG ACTGTGCTGC GGCAGACAAA TGACCAAATG ACTGAGGTCT GTGCCACGAC ATTCACAGAG AAGAATACAG TGGGCTTCCT AGATTACCCC TTCTGCAGTG GTACCTTTAA TGAAAGCAGA GTGAACCTCA CCATCCAAGG ACTGAGAGCT GTTGACACGG GACTGTACCT CTGCAAGGTG GAACTCATGT ACCCACCGCC ATACTTTGTG GGCATGGGCA ACGGGACGCA GATTTATGTC ATTGATCCAG AACCATGCCC GGATTCTGAC TTCCTCCTTT GGATCCTTGT CGCAGTTAGC TTGGGGTTGT TTTTTTACAG TTTCCTGGTC ACTGCTGTTT CTTTGAGCAA GATGACAAAT AGTAGAAGGA ACAGACTCCT TCAAAGTGAC TACATGAACA TGACTCCCCG GAGGCCTGGG CTCACTCGAA AGCCTTACCA GCCCTACGCC CCTGCCAGAG ACTTTGCAGC GTACCGCCCC TGA |
| 21 | | MASPLTRFLS LNLLLLGESI ILGSGEADIQ MTQSPASLST SLGETVTIQC QASEDIYSGL AWYQQKPGKS PQLLIYGASD LQDGVPSRFS GSGSGTQYSL KITSMQTEDE GVYFCQQGLT YPRTFGGGTK LELKGGGGSG GGGSGGGGSE VQLQQSGAEL VRPGTSVKLS CKVSGDTITF YYMHFVKQRP GQGLEWIGRI DPEDESTKYS EKFKNKATLT ADTSSNTAYL KLSSLTSEDT ATYFCIYGGY YFDYWGQGVM VTVSSTTTKP VLRTPSPVHP TGTSQPQRPE DCRPRGSVKG TGLDFACDIY IWAPLAGICV ALLLSLIITL ICKRGRKKLL YIFKQPFMRP VQTTQEEDGC SCRFPEEEEG GCELRAKFSR SAETAANLQD PNQLYNELNL GRREEYDVLE KKRARDPEMG GKQQRRRNPQ EGVYNALQKD KMAEAYSEIG TKGERRRGKG HDGLYQGLST ATKDTYDALH MQTLAPRGSG ATNFSLLKQA GDVEENPGPM ACLGLRRYKA |
| | | QLQLPSRTWP FVALLTLLFI PVFSEAIQVT QPSVVLASSH GVASFPCEYS PSHNTDEVRV TVLRQTNDQM TEVCATTFTE KNTVGFLDYP FCSGTFNESR VNLTIQGLRA VDTGLYLCKV ELMYPPPYFV GMGNGTQIYV IDPEPCPDSD FLLWILVAVS LGLFFYSFLV TAVSLSKMTN SRRNRLLQSD YMNMTPRRPG LTRKPYQPYA PARDFAAYRP |
| 22 | CD19BBz-CTC28 human | ATGGCCTTAC CAGTGACCGC CTTGCTCCTG CCGCTGGCCT TGCTGCTCCA CGCCGCCAGG CCGGACATCC AGATGACACA GACTACATCC TCCCTGTCTG CCTCTCTGGG AGACAGAGTC ACCATCAGTT GCAGGGCAAG TCAGGACATT AGTAAATATT TAAATTGGTA TCAGCAGAAA CCAGATGGAA CTGTTAAACT CCTGATCTAC CATACATCAA GATTACACTC AGGAGTCCCA TCAAGGTTCA GTGGCAGTGG GTCTGGAACA GATTATTCTC TCACCATTAG CAACCTGGAG CAAGAAGATA TTGCCACTTA CTTTTGCCAA CAGGGTAATA CGCTTCCGTA CACGTTCGGA GGGGGGACCA AGCTGGAGAT CACAGGTGGC GGTGGCTCGG GCGGTGGTGG GTCGGGTGGC GGCGGATCTG AGGTGAAACT GCAGGAGTCA GGACCTGGCC TGGTGGCGCC CTCACAGAGC CTGTCCGTCA CATGCACTGT CTCAGGGGTC TCATTACCCG ACTATGGTGT AAGCTGGATT CGCCAGCCTC CACGAAAGGG TCTGGAGTGG CTGGGAGTAA TATGGGGTAG TGAAACCACA TACTATAATT CAGCTCTCAA ATCCAGACTG ACCATCATCA AGGACAACTC CAAGAGCCAA GTTTTCTTAA AAATGAACAG TCTGCAAACT GATGACACAG CCATTTACTA CTGTGCCAAA CATTATTACT ACGGTGGTAG CTATGCTATG GACTACTGGG GCCAAGGAAC CTCAGTCACC GTCTCCTCAA CCACGACGCCAGCGCCGCGA CCACCAACAC CGGCGCCCAC CATCGCGTCG CAGCCCCTGT CCCTGCGCCC AGAGGCGTGC CGGCCAGCGG CGGGGGGCGC AGTGCACACG AGGGGGCTGG ACTTCGCCTG TGATATCTAC ATCTGGGCGC |
| | | CCTTGGCCGG GACTTGTGGG GTCCTTCTCC TGTCACTGGT TATCACCCTT TACTGCAAAC GGGGCAGAAA GAAACTCCTG TATATATTCA AACAACCATT TATGAGACCA GTACAAACTA CTCAAGAGGA AGATGGCTGT AGCTGCCGAT TTCCAGAAGA AGAAGAAGGA GGATGTGAAC TGAGAGTGAA GTTCAGCAGG AGCGCAGACG CCCCCGCGTA CAAGCAGGGC CAGAACCAGC TCTATAACGA GCTCAATCTA GGACGAAGAG AGGAGTACGA TGTTTTGGAC AAGAGACGTG GCCGGGACCC TGAGATGGGG GGAAAGCCGA GAAGGAAGAA CCCTCAGGAA GGCCTGTACA ATGAACTGCA GAAAGATAAG ATGGCGGAGG CCTACAGTGA GATTGGGATG AAAGGCGAGC GCCGGAGGGG CAAGGGGCAC GATGGCCTTT ACCAGGGTCT CAGTACAGCC ACCAAGGACA CCTACGACGC CCTTCACATG CAGGCCCTGC CCCCTCGCGG ATCTGGCGCC ACCAATTTCA GCCTGCTGAA ACAGGCTGGC GACGTGGAAG AGAACCCTGG ACCTATGGCC TGCCTGGGCT TCCAGAGACA CAAGGCCCAA CTGAACCTGG CCACCAGAAC ATGGCCCTGT ACTCTGCTGT TCTTCCTGCT GTTCATCCCC GTGTTCTGCA AGGCCATGCA TGTGGCTCAG CCAGCTGTGG TGCTGGCCTC TTCTAGAGGA ATCGCCAGCT TCGTGTGCGA GTACGCCTCT CCTGGAAAGG CCACTGAAGT GCGCGTGACC GTTCTGAGAC AGGCCGATAG CCAAGTGACC GAAGTGTGCG CCGCCACCTA CATGATGGGC AACGAGCTGA CCTTCCTGGA CGACAGCATC TGTACCGGCA CCAGCAGCGG CAATCAAGTG AACCTGACCA TCCAGGGCCT GAGAGCCATG GATACCGGCC TGTACATCTG CAAGGTGGAA CTGATGTACC CTCCTCCTTA CTACCTCGGC ATCGGCAACG GCACCCAGAT CTACGTGATC GACCCTGAGC CTTGTCCTGA CAGCGACTTT CTGCTGTGGA TTCTGGCCGC CGTGTCCAGC GGCCTGTTCT TCTACTCTTT TCTGCTGACC GCTGTGTCCC TGAGCAAGAT GGTCCGAAGC |
| | | AAGCGGAGCA GACTGCTGCA CAGCGACTAC ATGAACATGA CCCCTAGACG GCCCGGACCT ACCAGAAAGC ACTACCAGCC TTACGCTCCT CCTAGAGACT TCGCCGCCTA CAGAAGCTGA |
| 23 | | MALPVTALLL PLALLLHAAR PDIQMTQTTS SLSASLGDRV TISCRASQDI SKYLNWYQQK PDGTVKLLIY HTSRLHSGVP SRFSGSGSGT DYSLTISNLE QEDIATYFCQ QGNTLPYTFG GGTKLEITGG GGSGGGGSGG GGSEVKLQES GPGLVAPSQS LSVTCTVSGV SLPDYGVSWI RQPPRKGLEW LGVIWGSETT YYNSALKSRL TIIKDNSKSQ VFLKMNSLQT DDTAIYYCAK HYYYGGSYAM DYWGQGTSVT VSSTTTPAPR PPTPAPTIAS QPLSLRPEAC RPAAGGAVHT RGLDFACDIY IWAPLAGTCG VLLLSLVITL YCKRGRKKLL YIFKQPFMRP VQTTQEEDGC SCRFPEEEEG GCELRVKFSR SADAPAYKQG QNQLYNELNL GRREEYDVLD KRRGRDPEMG GKPRRKNPQE GLYNELQKDK MAEAYSEIGM KGERRRGKGH DGLYQGLSTA TKDTYDALHM QALPPRGSGA TNFSLLKQAG DVEENPGPMA CLGFQRHKAQ LNLATRTWPC TLLFFLLFIP VFCKAMHVAQ PAVVLASSRG IASFVCEYAS PGKATEVRVT VLRQADSQVT EVCAATYMMG NELTFLDDSI CTGTSSGNQV NLTIQGLRAM DTGLYICKVE LMYPPPYYLG IGNGTQIYVI DPEPCPDSDF LLWILAAVSS GLFFYSFLLT AVSLSKMVRS KRSRLLHSDY MNMTPRRPGP TRKHYQPYAP PRDFAAYRS |

It is understood that although the present application refers to "comprising a gene sequence/amino acid sequence of a particular sequence number" or "having a gene sequence/amino acid sequence of a particular sequence number," it is intended that some sequences may have deletions, modifications, substitutions, or additions to the gene sequence/amino acid sequence, as long as the sequence has the same or equivalent function as that comprised in the gene sequence/amino acid sequence of that sequence number. In addition, gene sequences and sequences of bases may be used interchangeably in this application.

For example, it is obvious that if it has the same or corresponding function as the CTLA4-CD28 chimeric protein, the chimeric antigen receptor, or the construct, it is also within the scope of the present invention that a meaningless sequence is added to or at the end of the sequence of the sequence number, or a part of the sequence of the sequence number or at the end is deleted.

Homology and identity refer to the degree to which two given sequences are related and can be expressed as a percentage. The terms homology and identity are often used interchangeably.

Whether any two sequences have homology or identity can be determined using known computer algorithms such as the "FASTA" program using default parameters as in Pearson et al (1988) [Proc. Natl. Acad. Sci. USA 85: 2444]. Alternatively, it can be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (This includes the GCG program package (Devereux, J., et al, Nucleic Acids Research 12: 387 (1984)), BLASTP, BLASTN, FASTA (Altschul, S. F., et al, J Molec Biol 215: 403 (1990)); Guide to Huge Computers, Martin J. Bishop, ed., Academic Press, San Diego, 1994, and Carrillo et al. (1988) SIAM J Applied Math 48: 1073). For example, the homology or identity of sequences can be determined using BLAST from the National Center for Biotechnology Information (NCBI) of the United States, or ClustalW.

Another aspect provides an immune cell into which the construct or a vector comprising the construct has been introduced.

In some embodiments, T-cells transduced using the viral or non-viral vector can be provided.

Another aspect provides an immune cell comprising a protein expressed from said construct or a vector comprising said construct.

In some embodiments, the immune cells may be T cells, NK cells, NKT cells, macrophages, or combinations thereof, but are not limited to.

In some embodiments, the immune cells may be CD4 T cells, CD8 T cells, or combinations thereof, but are not limited to.

T-cells comprising the CTLA4-CD28 chimeric gene and first-generation CAR or 4-1BB-based second-generation CAR have excellent cancer therapeutic efficacy and can be easily eliminated by α-CTLA4, proving their safety and usefulness in T-cell immunotherapy.

Another aspect provides an immune cell comprising:
(i) a first gene encoding a fusion protein (CTLA4-CD28 fusion protein) comprising a CTLA4 protein or a domain thereof; and CD28 or a domain thereof; and
(ii) a second gene encoding a chimeric antigen receptor comprising an antigen binding domain; an extracellular spacer domain; a transmembrane domain; and an intracellular signaling domain.

In some embodiments, the first gene and the second gene may each be independently introduced into the immune cell simultaneously, sequentially, or in reverse order.

Another aspect provides an immune cell expressing both:
(i) a fusion protein (CTLA4-CD28 fusion protein) comprising a CTLA4 protein or a domain thereof; and CD28 or a domain thereof; and
(ii) a chimeric antigen receptor comprising an antigen binding domain; an extracellular spacer domain; a transmembrane domain; and an intracellular signaling domain.

Another aspect provides a composition comprising the construct, a vector comprising the construct, or the immune cell. Still another aspect provides a composition comprising the construct, a vector comprising the construct, or the immune cells, and a pharmaceutically acceptable carrier.

In some embodiments, the construct, the vector, the immune cell, or the composition may be for the prevention or treatment of a disease (e.g., cancer).

Another aspect provides the use of the construct, the vector, the immune cell, or the composition for the manufacture of a medicament for the prevention or treatment of a disease (e.g., cancer).

As used herein, the term "disease" may refer to any pathologic condition, in particular cancer, infectious disease, inflammatory disease, degenerative disease, apoptosis-related disease, and graft rejection.

As used herein, the term "treatment" refers to or includes alleviation, inhibition of progression, or prevention of a disease, disorder, or condition, or one or more symptoms thereof, and the term "active ingredient" or "pharmaceutically effective amount" may refer to any amount of a composition used in practicing the invention provided herein that is sufficient for alleviation, inhibition of progression, or prevention of a disease, disorder, or condition, or one or more symptoms thereof.

As used herein, the terms "administering," "introducing," and " transplanting" are used interchangeably and may refer to the placement of a composition according to a specific embodiment into a subject using a method or a route for causing at least partial localization on a desired portion. At least a portion of the cells or cellular components of a composition according to one embodiment may be administered by any suitable route that delivers the cells or cellular components to a desired location within a living subject. **The** survival period of the cells after administration to the subject may be as short as several hours, such as 24 hours, to as long as several days, or to as long as several years.

The administration may be in combination with an additional anticancer agent. Examples of additional anticancer agents may include alkylating agents, antimetabolites, spindle inhibitor plant alkaloids, cytostatic/antitumor antibiotics, topoisomerase inhibitors, antibodies, photosensitizers, and kinase inhibitors. Examples of the above anticancer agents can include compounds used in targeted therapies and conventional chemotherapy. Further, examples of antibodies may include alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemtuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab, and visilizumab.

The cancer or carcinoma is not particularly limited and includes solid and hematologic cancers. Specifically, the cancer or carcinoma includes stomach cancer, lung cancer, breast cancer, ovarian cancer, liver cancer, bronchial cancer, nasopharyngeal cancer, laryngeal cancer, pancreatic cancer, bladder cancer, colon cancer, colorectal cancer, pancreatic cancer, cervical cancer, brain cancer, prostate cancer, bone cancer, skin cancer, thyroid cancer, parathyroid cancer, kidney cancer, esophageal cancer, biliary tract cancer, testicular cancer, rectal cancer, head and neck cancer, cervical spine cancer, ureteral cancer, osteosarcoma, neuroblastoma, melanoma, fibrosarcoma, rhabdomyosarcoma, astrocytoma, neuroblastoma or glioma, and more preferably, colon cancer, ovarian cancer, stomach cancer, pancreatic cancer, breast cancer, and the like.

The composition according to the invention may further comprise a pharmaceutically acceptable carrier. As a pharmaceutically acceptable carrier, a binder, a glidant, a disintegrant, an excipient, a solubilizer, a dispersant, a stabilizer, a suspending agent, a pigment, a flavor, and the like may be used for oral administration. A buffer, a preserving agent, a pain-relieving agent, a solubilizer, an isotonic agent, a stabilizer, and the like may be used in admixture for injections. A base, an excipient, a lubricant, a preserving agent, and the like may be used for topical administration. The pharmaceutical composition according to the present invention may be formulated in various ways by mixing with pharmaceutically acceptable carriers as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. In addition, the anticancer composition may typically include a surfactant to facilitate migration across membranes. Such surfactant may include those derived from steroids, cationic lipids such as N-[1-(2,3-dioleoyl)propyl-N,N,N-trimethylammonium chloride (DOTMA), or various compounds such as cholesterol hemisuccinate and phosphatidyl glycerol.

Another aspect provides a method for the prevention or treatment of a disease (e.g., cancer), comprising the step of administering the construct, the vector, the immune cell, or the composition to a subject in need thereof.

For example, the method may include, but is not limited to, inhibiting cancer growth.

In some embodiments, the composition may be administered in a pharmaceutically effective amount to treat cancer cells or their metastases, or to inhibit the growth of cancer. The amount may vary depending on various factors, including the type of cancer, the age and weight of the patient, the nature and severity of symptoms, the type of current therapy, the number of treatments, the dosage form and route of administration, and can be readily determined by one of ordinary skill in the art. The composition according to the present invention may be administered together with or sequentially with the above pharmacological or physiological components. The composition may also be administered in combination with additional conventional therapeutic agents and may be administered sequentially or simultaneously with conventional therapeutic agents. Such administration may be single or multiple administrations. Taking all of the above factors into consideration, it is important to administer an amount that can obtain the maximum effect with the minimum amount without side effects, which can be readily determined by a person skilled in the art.

Another aspect provides a method for producing an immune cell expressing (a) a fusion protein (CTLA4-CD28 fusion protein) comprising a CTLA4 protein or a domain thereof; and CD28 or a domain thereof and (b) a chimeric antigen receptor comprising an antigen binding domain; an extracellular spacer domain; a transmembrane domain; and an intracellular signaling domain, the method comprising the step of introducing into an immune cell:
(i) a first gene encoding the CTLA4-CD28 fusion protein; and
(ii) a second gene encoding the chimeric antigen receptor.

In some embodiments, said step may comprise, but is not limited to, introducing into the immune cell the construct, or a vector comprising the construct.

In some embodiments, the step may include introducing the first gene and the second gene each independently into immune cells simultaneously, sequentially, or in reverse order, but are not limited to. In this case, independently introducing the first gene and the second gene can be performed according to methods known in the art. For example, a vector comprising the first gene and a vector comprising the second gene can be used independently, but is not limited thereto.

### [Form for practicing the invention]

Preferred embodiments of the present invention are described below to facilitate understanding of the present invention. However, the following embodiments are provided only to facilitate understanding of the invention, and the invention is not limited by the following embodiments. The embodiments are subject to various modifications, and the embodiments are not limited to those disclosed herein, but may be implemented in various forms.

### Example 1. Anti-cancer efficacy of CTC28-loaded first-generation CAR T cells

### 1.1. Construction of CTC28-loaded 1^{st} generation CAR retrovirus and T cell transduction

The main preclinical animal model currently used to evaluate the anticancer efficacy of CAR T cells is a model where human CAR T cells are administered to immunodeficient mice inoculated with human tumors. However, this model has the disadvantage of being difficult to verify the immunological efficacy of CAR T cells due to the absence of immune cells in the body. Therefore, the present inventors sought to first verify the concept of enhanced anticancer efficacy of CAR T cells by introducing CTC28 using normal mice with preserved immune function. For this purpose, mouse CAR T cells targeting CD19 were constructed, and a mouse syngeneic tumor model was established to test the efficacy of these CAR T cells against CD19-positive mouse lymphoma cells.

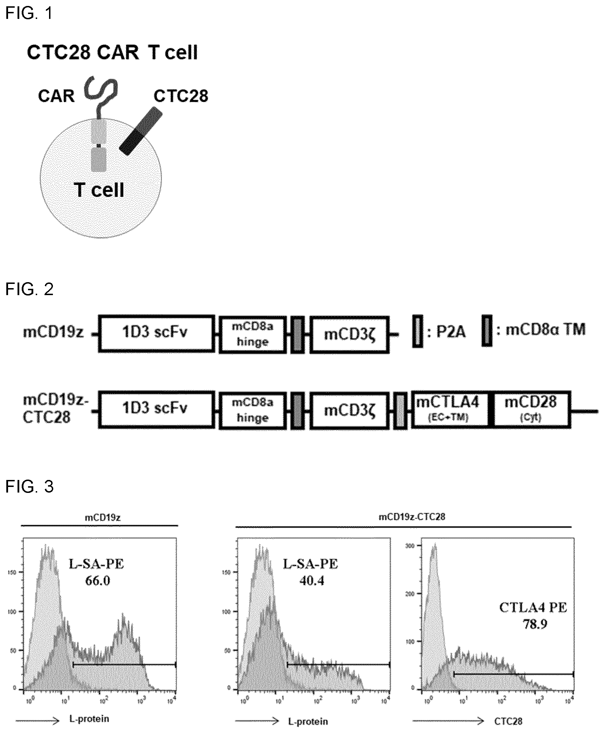

Retroviruses for CAR expression were produced to prepare CAR T cells co-transduced with CTC28 (see FIG. 1).

Specifically, a first-generation CAR gene (mCD19z) was constructed by linking the antigen-binding site of the 1D3 antibody, which is an antibody against mouse CD19, with the intracellular signaling domain of the mouse T cell receptor signaling subunit (CD3zeta), and this was cloned into a retrovirus vector.

The protein coding region of the first-generation mouse CAR (mCD19z) consists of the signal peptide of mouse immunoglobulin kappa chain, scFv of anti-mouse CD19 antibody (1D3), mouse CD8 extracellular and transmembrane domains, and mouse CD3zeta intracellular domain. These CAR cDNAs were synthesized by commissioning DNA synthesis from Bioneer Korea and IDT (Integrated DNA Technologies) USA. The retroviral vector for CAR expression was cloned from the pMSCV-puro retroviral vector (Clontech, USA) by replacing the PuroR gene site under the PGK promoter with the constructed CAR cDNAs. The mouse CTLA4-CD28 chimera (CTC28) protein coding region was constructed as previously reported (Blood 2012;119(24):5678-87), which consists of the extracellular and transmembrane domains of mouse CTLA4 linked to the intracellular domain of mouse CD28. The schematic is shown in FIG. 2.

For the production of an ecotrophic retrovirus for mouse CAR expression, the mouse CAR retroviral vector and VSV-G envelope expression plasmid (pMD2.G, Addgene plasmid #12259) were transfected into Phoenix GP cell lines with Lipofectamin 3000 (Invitrogen). 48 hours later, the Phoenix Eco cell lines were transduced again with VSV-G pseudotyped retroviruses secreted into the culture supernatant. After 3-5 days, cells stably expressing CAR protein on the cell surface were isolated using a cell sorter (FACS Aria, Becton Dickinson) and used as an ecotrophic retrovirus production cell line. The retroviral culture supernatant produced from this cell line was concentrated 5-10-fold using a centrifugation filter (Amicon Ultra-100 kDa cut-off, Millipore, USA) and used for the production of mouse CAR T cells.

Flow cytometric analysis (FACS, fluorescence activated cell sorter) was performed to determine the expression of CAR (L-protein) and CTC28 on transduced T cells, and the results are shown in FIG. 3.

FIG. 1 is a schematic diagram showing the dual gene-engineered T cell using the CTLA-4 mutant and the chimeric antigen receptor.

FIG. 2 shows the gene construct structures of the 1^{st} generation CAR (mCD19z) and the 1^{st} generation CAR and CTLA4-CD28 (mCD19z-CTC28).

FIG. 3 shows graphs analyzing the expression of CAR and CTLA4 proteins on the surface of T cells transduced with the 1^{st} generation CAR (mCD19z) or the 1^{st} generation CAR and CTC28 (mCD19z-CTC28).

As shown in FIG. 3, T cells transduced with first-generation CAR (mCD19z) showed 66.0% CAR-positive cells, while T cells transduced with first-generation CAR and CTLA4-CD28 (mCD19z-CTC28) showed 40.4% CAR-positive cells and 78.9% CTC28-positive cells, confirming good CAR and CTC28 expression in all cases.

### 1.2. Anticancer efficacy of CTC28-loaded 1^{st} generation CAR T cells - in vitro

To evaluate the *in vitro* anticancer efficacy of T cells co-expressing first-generation CAR and CTC28, the tumor killing ability of the produced first-generation CAR and CTC28 co-expressing T cells against A20 cells, a mouse B cell lymphoma cell line, was analyzed.

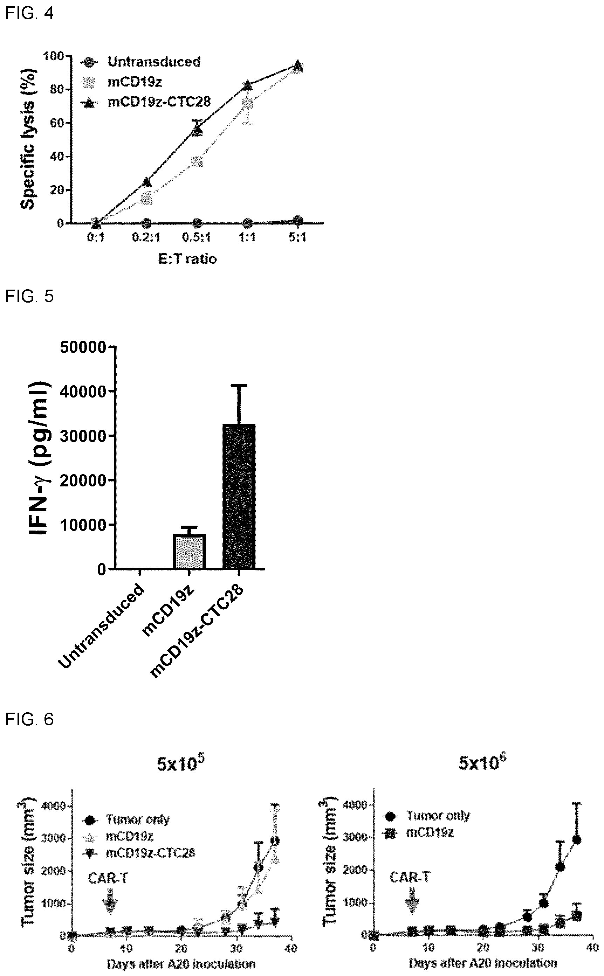

Specifically, A20 (BALB/c-derived lymphoma cell line) was purchased from ATCC (USA), and a GFP-luciferase gene-transfected cell line (A20-Luc) was prepared by transducing A20 cells with a retrovirus for GFP-Luc expression (pMP-LucGFP) and isolating GFP-positive cells using a cell sorter (FACS Aria, Becton Dickinson). For measuring the tumor killing ability of CAR T cells, proliferated CAR T cells after retroviral transduction (Effector cell, 1.2×10³ to 7.5×10⁵ cells/100µl/well) were added to A20-Luc cells (Target cell, 3×10⁴ cells/50µl/well) at various ratios (0.04 to 25:1) and co-cultured overnight in 96 well plates. Then, D-Luciferin (600µg/ml, Promega) 50µl was added and incubated at 37°C for 10 minutes to induce luciferase enzyme activity in surviving A20-Luc cells. The luminescence of these cells was measured using a Luminometer (Tecan), and the tumor killing ability of CAR T cells was measured by calculating the survival rate of tumor cells compared to the luminescence of A20-Luc cells not treated with CAR T cells. The results are shown in FIG. 4.

Additionally, to evaluate the cytokine production ability of T cells co-expressing first-generation CAR and CTC28, the amount of IFN-γ secreted when the produced first-generation CAR and CTC28 co-expressing T cells were co-cultured with A20 cells was measured.

Specifically, CAR T cells and target cells A20 were mixed in equal numbers (3X10⁴cells) and co-cultured in 96 well plates for 24 hours, and the culture supernatant was harvested. The amount of IFN-γ secreted into the supernatant was measured by ELISA (mouse IFNγ ELISA kit, BD Biosciences), and the results are shown in FIG. 5.

FIG. 4 shows the tumor killing ability of T cells transduced with the 1^{st} generation CAR (mCD19z) or the 1^{st} generation CAR and CTC28 (mCD19z-CTC28) against A20 cells.

FIG. 5 illustrates a graph showing the amount of IFN-γ secreted by T cells transduced with the 1^{st} generation CAR (mCD19z) or the 1^{st} generation CAR and CTC28 (mCD19z-CTC28) when co-cultured with A20 cells.

As shown in FIG. 4, in co-culture with A20 cells, the first generation CAR T cells loaded with CTC28 exhibited similar or slightly enhanced tumor killing capacity compared to first generation CAR T cells.

In contrast, as shown in FIG. 5, the amount of IFN-γ secreted by the first generation CAR T cells loaded with CTC28 was significantly increased compared to the first generation CAR T cells, confirming that expression of CTC28 significantly increased the cytokine activity of CAR T cells.

### 1.3. Anti-cancer efficacy of CTC28-loaded 1^{st} generation CAR T cells - in vivo

To evaluate the *in vivo* anti-cancer efficacy of T cells co-expressing first-generation CAR and CTC28, CAR T cells were intravenously injected into Balb/C mice subcutaneously inoculated with A20 cells, and tumor growth was measured over time.

Specifically, BALB/c mice were purchased from Orient Bio (Seongnam, Republic of Korea), housed in specific pathogen-free (SPF) animal facilities at Seoul National University College of Medicine (Seoul, Republic of Korea), and maintained according to institutional animal care and use committee (IACUC) guidelines. To simultaneously evaluate the *in vivo* efficacy and toxicity of CAR-T cells in a syngeneic mouse model, BALB/c mice were subcutaneously injected with A20 cells (5×10⁶ cells per mouse). After 7 days, low-dose whole-body irradiation (2.5Gy) was applied for lymphodepletion. Two days after irradiation, CAR T cells proliferated for 4-5 days after retroviral transduction (1×10⁶ cells or 5×10⁶ cells per mouse) were intravenously injected. Subsequently, the *in vivo* efficacy of CAR-T was evaluated by measuring the tumor size using a caliper twice a week, and the results are shown in FIG. 6.

FIG. 6 illustrates graphs showing the changes in tumor size in mice injected with T cells transduced with the 1^{st} generation CAR (mCD19z) or the 1^{st} generation CAR and CTC28 (mCD19z-CTC28) (Tumor size = (long axis)² x short axis/2).

As shown in Figure 6, for first-generation CAR T cells, significant tumor growth inhibitory effects could be observed when 5×10⁶ CAR T cells were administered, but almost no antitumor effect was observed when 5×10⁶ CAR T cells were administered. However, in the case of first generation CAR T cells loaded with CTC28, even when 5×10⁶ cells were administered, significant tumor suppression effects comparable to those when 5×10⁶ first-generation CAR T cells were administered were observed, confirming that the antitumor effect of CTC28 CAR T cells is approximately 10 times more potent than first-generation CAR T cells.

These results demonstrate that co-expression of CTC28 significantly enhances antitumor activity against first-generation CARs.

### Example 2. Anti-cancer efficacy of CTC28-loaded 2^{nd} generation CAR T cells

### 2.1. Construction of CTC28-loaded 2^{nd} generation CAR retrovirus and T cell transduction

CAR T cells currently in clinical use as therapeutic agents are second-generation CAR T cells that contain either CD28 or 41BB intracellular signaling domain on the intracellular signaling domain of CAR molecules (e.g., Axicabtagene ciloleucel is a CD28-based second-generation CAR T cell therapy and Tisagenlecleucel is a 41BB-based second-generation CAR T cell therapy). Therefore, to determine whether co-expression of CTC28 can enhance the efficacy of second-generation CAR T cells, CD28-based and 4-1BB-based second-generation mouse CAR T cells loaded with CTC28 were constructed.

Specifically, the protein coding region of CD28-based second-generation mouse CAR (mCD1928z) consists of rat immunoglobulin kappa chain signal peptide, anti-mouse CD19 antibody (1D3) scFv, mouse CD28 extracellular domain-transmembrane domain-intracellular domain, and mouse CD3zeta intracellular region according to previously reported sequences (GenBank HM754222.1, Blood 2010;116(20): 4099-4102).

The protein coding region of 4-1BB-based second-generation mouse CAR (mCD19BBz) consists of mouse CD8 signal peptide, anti-mouse CD19 antibody (1D3) scFv, mouse CD8 extracellular domain and transmembrane domain, human 4-1BB intracellular domain, and mouse CD3zeta intracellular domain. For the 4-1BB intracellular domain, the human 4-1BB intracellular domain was used according to previous reports that human 4-1BB sequences are more functional than mouse 4-1BB sequences in mouse CAR T cells (JCI Insight. 2018;3(18):e121322). These CAR cDNAs were synthesized by commissioning DNA synthesis from Bioneer Korea and IDT (Integrated DNA Technologies) USA. The retroviral vector for CAR expression was constructed by the same method as Example 1.1 above, by cloning the pMSCV-puro retroviral vector (Clontech, USA) with the PuroR gene site under the PGK promoter replaced with the constructed CAR cDNA. The mouse CTLA4-CD28 chimera (CTC28) protein coding region and mouse CAR T cell construction using it were performed by the same method as Example 1.1 above.

### 2.2. Anti-cancer efficacy of CD28-based 2^{nd} generation CAR T cells loaded with CTC28

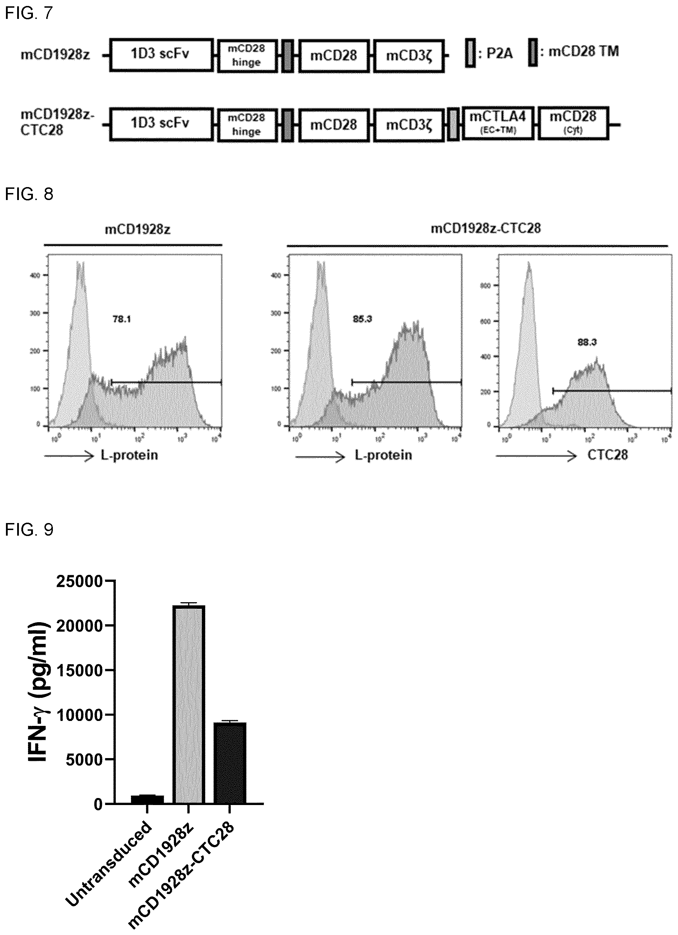

First, to verify the CTC28 co-loading effect in CD28-based second-generation CAR T cells, retroviruses expressing gene constructs with schematics as shown in FIG. 7 were transduced into mouse T cells.

Flow cytometry (FACS, fluorescence activated cell sorter) was performed to determine the expression of CAR (L-protein) and CTC28 in the transduced T cells in the same manner as in Example 1.2 above, and the results are shown in FIG. 8.

To evaluate the *in vitro* anti-cancer efficacy of the second generation CAR and CTC28 co-expressing T cells, the amount of IFN-γ secreted by the prepared second generation CAR and CTC28 co-expressing T cells when co-cultured with A20 tumor cells was measured using the same method as in Example 1.2 above, and the results are shown in FIG. 9.

To evaluate the *in vivo* anti-cancer efficacy of T cells co-expressing second-generation CAR and CTC28, CAR T cells were intravenously injected into Balb/C mice subcutaneously inoculated with A20 cells, and tumor growth was measured over time.

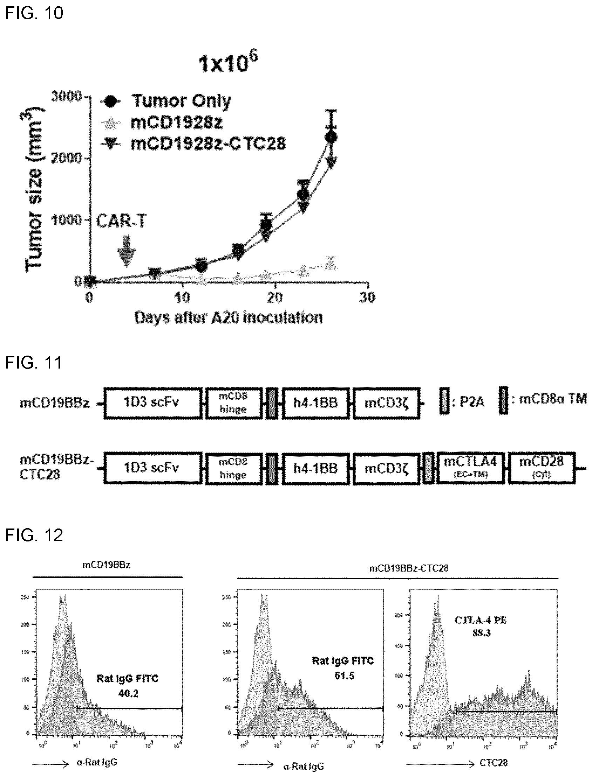

Specifically, Balb/C mice were injected subcutaneously with a low dose of total body irradiation (3Gy) and A20 cells (5X10⁶ cells per mouse) for lymphodepletion. 3 days later, CAR T cells were injected intravenously, and tumor growth was measured over time using the same method as in Example 1.3 above, and the results are shown in FIG. 10.

FIG. 7 shows the gene construct structures of the CD28-based 2^{nd} generation CAR (mCD1928z) and the 2^{nd} generation CAR and CTLA4-CD28 (mCD1928z-CTC28).

FIG. 8 shows graphs analyzing the expression of CAR and CTLA4 proteins on the surface of T cells transduced with the CD28-based 2^{nd} generation CAR (mCD1928z) or the 2^{nd} generation CAR and CTC28 (mCD1928z-CTC28).

FIG. 9 illustrates a graph showing the amount of IFN-γ secreted by T cells transduced with the CD28-based 2^{nd} generation CAR (mCD1928z) or the 2^{nd} generation CAR and CTC28 (mCD1928z-CTC28) when co-cultured with A20 cells.

FIG. 10 illustrates a graph showing the changes in tumor size in mice injected with T cells transduced with the CD28-based 2^{nd} generation CAR (mCD1928z) or the 2^{nd} generation CAR and CTC28 (mCD1928z-CTC28) (Tumor size = (long axis)² x short axis/2).

As shown in FIG. 8, T cells transduced with second-generation CAR (mCD1928z) showed 78.1% CAR-positive cells, while T cells transduced with second-generation CAR and CTLA4-CD28 (mCD1928z-CTC28) showed 85.3% CAR-positive cells and 88.3% CTC28-positive cells, confirming good CAR and CTC28 expression in all cases.

As shown in FIG. 9, contrary to the pattern observed in first-generation CAR T cells, the IFN-γ secretion of CD28-based second-generation CTC28 CAR T cells was significantly lower compared to second-generation CAR T cells.

As shown in FIG. 10, in a mouse tumor model, administration of the same number of second-generation CTC28 CAR T cells (1x10⁶) as the number of CD28-based second-generation CAR T cells with significant antitumor effects (1x10⁶) resulted in a significant decrease in antitumor potency.

Through this, it was observed that for CD28-based second-generation CAR T cells, unlike first-generation CAR T cells, co-expression of CTC28 actually inhibits the anticancer efficacy of CAR T cells.

Therefore, this suggests that the increase in anticancer efficacy of CAR T cells by CTC28 co-loading may not be seen for all CAR types, but may only be achieved for certain types of CAR.

### 2.3. Anti-cancer efficacy of 41BB-based 2^{nd} generation CAR T cells loaded with CTC28

Next, to verify the CTC28 co-loading effect in 4-1BB-based second-generation CAR T cells, retroviruses expressing gene constructs with schematics as shown in FIG. 11 were transduced into mouse T cells.

Flow cytometric analysis (FACS, fluorescence activated cell sorter) was performed to confirm the expression of CAR (α-Rat IgG) and CTC28 on transduced T cells, and the results are shown in FIG. 12.

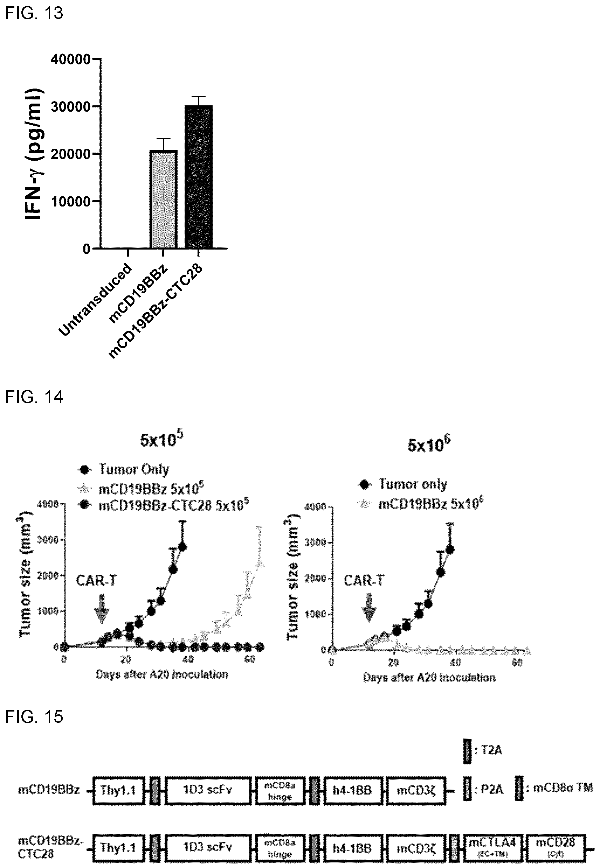

To evaluate the *in vitro* anticancer efficacy of T cells co-expressing 4-1BB-based second-generation CAR and CTC28, the amount of IFN-γ secreted from the prepared second generation CAR and CTC28 co-expressing T cells when co cultured with A20 tumor cells was measured by the same method as in Example 1.2 above, and the results are shown in FIG. 13.

To evaluate *the in vivo* anti-cancer efficacy of T cells co-expressing 41BB-based second-generation CAR and CTC28, CAR T cells were intravenously injected into Balb/C mice subcutaneously inoculated with A20 cells, and tumor growth was measured over time.

Specifically, Balb/C mice were injected subcutaneously with A20 cells (5X10⁶ cells per mouse) and 12 days later were irradiated with a low dose of total body irradiation (2.5 Gy) for lymphodepletion. Two days later, CAR T cells (1X10⁶ cells or 5X10⁶ cells per mouse) were injected intravenously, and tumor growth was measured over time using the same method as in Example 1.3 above, and the results are shown in FIG. 14.

FIG. 11 shows the gene construct structure of the 41BB-based 2^{nd} generation CAR (mCD19BBz) and the 2^{nd} generation CAR and CTLA4-CD28 (mCD19BBz-CTC28).

FIG. 12 illustrates graphs analyzing the expression of CAR and CTLA4 proteins on the surface of T cells transduced with the 41BB-based 2^{nd} generation CAR (mCD19BBz) or the 2^{nd} generation CAR and CTC28 (mCD19BBz-CTC28).

FIG. 13 illustrates a graph showing the amount of IFN-γ secreted by T cells transduced with the 41BB-based 2^{nd} generation CAR (mCD19BBz) or the 2^{nd} generation CAR and CTC28 (mCD19BBz-CTC28) when co-cultured with A20 cells.

FIG. 14 illustrates graphs showing the changes in tumor size in mice injected with T cells transduced with the 41BB-based 2^{nd} generation CAR (mCD19BBz) or the 2^{nd} generation CAR and CTC28 (mCD19BBz-CTC28) (Tumor size = (long axis)² x short axis/2).

As shown in Figure 12, T cells transduced with 4-1BB-based second-generation CAR (mCD1928z) showed 40.2% CAR-positive cells, while T cells transduced with second-generation CAR and CTLA4-CD28 (mCD1928z-CTC28) showed 61.5% CAR-positive cells and 88.3% CTC28-positive cells, confirming good CAR and CTC28 expression in all cases.

As shown in Figure 13, it was confirmed that the IFN-γ production of 4-1BB-based second-generation CTC28 CAR T cells was increased compared to second-generation CAR (4-1BB) T cells.

As shown in Figure 14, in the mouse tumor model, even at cell doses (5×10⁵) where 4-1BB-based second-generation CAR T cells could not completely eliminate tumors and tumors recurred, 4-1BB-based second-generation CTC28 CAR T cells showed efficacy in completely eliminating tumors, demonstrating that CTC28 co-introduction greatly enhances the efficacy of CAR T cells in 4-1BB-based CAR T cells.

Through this, it was confirmed that the efficacy of CTC28 co-loading varies depending on the type of intracellular signaling domain of the CAR construct. Specifically, the efficacy of CTC28 CAR T cells was enhanced in the case of first-generation CAR T cells and 4-1BB-based second-generation CAR T cells.

These results demonstrate that the addition of CTC28 to 4-1BB-based second-generation CAR T cells used in clinical practice can lead to the production of CAR T cells with much improved function.

### Example 3. Functional improvement and mechanism analysis of CD4 and CD8 T cell combination therapy in 41BB-based CAR T cell therapy with CTC28

### 3.1. Activation of CTC28-loaded CD4 and CD8 CAR T cells - in vitro

Based on previous research on classical T cell therapy loaded with CTC28, where the functional enhancement effect of CTC28 appeared more strongly in CD4 T cells compared to CD8 T cells among T cell subsets. Treatment effects were maximized when these CTC28 CD4 T cells and CTC28 CD8 T cells were combined and injected in equal numbers (Blood 2012;119(24):5678-87). This CAR T cell study sought to determine whether such CD4 bias of CTC28 effects and enhancement effects of CTC28 in CD4 and CD8 T cell combination therapy are observed.

Specifically, to analyze the mechanism of enhanced function of 4-1BB-based CTC28 CAR T cells by dividing them into CD4 and CD8 T cells, a retroviral vector for CAR expression with an additional Thy1.1 marker for tracking mouse CAR T cells in the blood was constructed. The Thy1.1 protein coding region and the T2A-CAR or T2A-CARP2A-CTC28 protein coding region were amplified by PCR, and each amplified fragment was subjected to blunt-end ligation and then cloned into the pMSCV-purovector. The mouse CTLA4-CD28 chimera (CTC28) protein coding region and the production of mouse CAR T cells using it were performed by the same method as in Example 1.1 above. The schematic diagram is shown in FIG. 15.

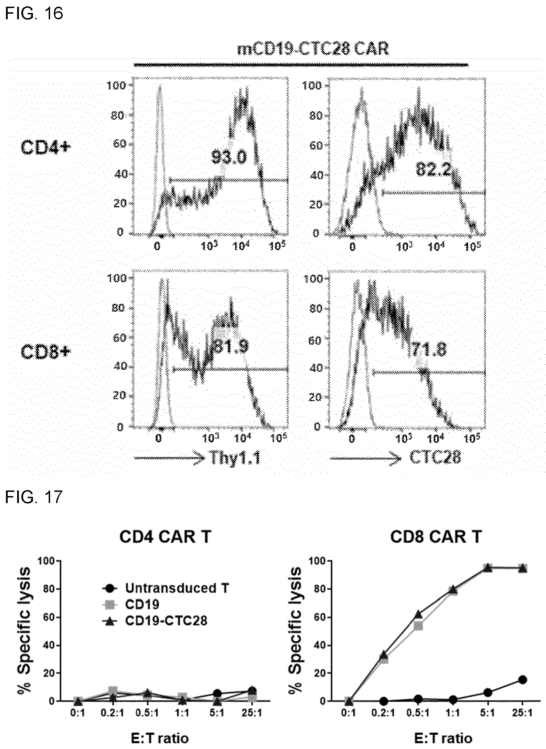

Specifically, for mouse CAR T cells, spleen and lymph node cells from normal Balb/C mice were stained with anti-CD4 microbead or anti-CD8 microbead (Miltenyi Biotech), respectively, and then CD4 T cells and CD8 T cells were isolated and purified using MACS LS column (Miltenyi Biotech). The cells were then added to 24 well plates coated with anti-CD3 antibody (145-2C11, 10µg/ml, BioXcell) together with anti-CD28 antibody (37.51, 2µg/ml, BD Biosciences) to activate each T cell. T cells activated for 24 hours were transduced with retroviruses by centrifugation at 24°C, 2500rpm for 90 minutes in the presence of 6µg/ml polybrene (Sigma Aldrich) together with concentrated retrovirus. The retroviral transduction process through centrifugation was repeated once on the same day. Subsequently, T cells were cultured for 48 hours in the presence of mouse IL-2 (30U/ml, Gibco). These retroviral-transduced T cells were washed twice, then fresh culture medium containing mouse IL-2 (20U/ml) was added and proliferated for 2-3 days for use as CAR T cells. After CAR T cell construction, expression of CAR protein on the cell surface was measured by flow cytometry (FACS-Canto II, BD Biosciences) after staining with biotinylated protein L (Thermo) and PE-labeled streptavidin, or FITC-labeled anti-rat IgG Fab fragment (Jackson ImmunoResearch). Expression of CTC28 and Thy1.1 was analyzed by flow cytometry after staining with fluorescence-labeled anti-CTLA4 antibody (UC10-4B9, Biolegend) and anti-Thy1.1 antibody (OX-7, Biolegend), respectively. The results are shown in FIG. 16.

Specifically, to determine the killing ability of CAR-transduced CD4 and CD8 T cells through co-culture with A20 cells, the tumor killing ability of CAR T cells was measured by calculating the survival rate of tumor cells compared to the luminescence of A20-Luc cells not treated with CAR T cells by the same method as Example 1.2 above, and the results are shown in FIG. 17.

Specifically, to evaluate the cytokine production ability of CAR-transduced CD4 and CD8 T cells, the amounts of IFN-γ, IL-2, and TNF-α secreted when CAR-transduced CD4 and CD8 T cells were co-cultured with A20 cells were measured by the same method as Example 1.2 above.

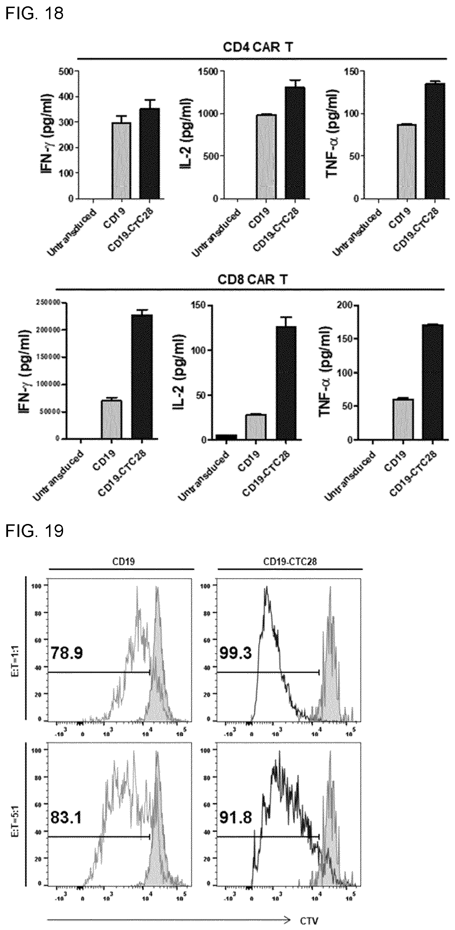

Additionally, to analyze the proliferation ability of CAR-transduced T cells, a dye dilution assay was performed to measure the degree of reduction in fluorescence intensity of dye stained in cells as CAR T cells stained with CTV dye proliferated. Specifically, CAR-T cells were labeled with CTV (2.5µM, eBioscience) and then mixed in equal numbers (3×10⁴ cells) with target cells (A20 cells) and added to 96 well plates. After 48 hours or 72 hours, cells were harvested and stained with anti-Thy1.1 antibody (OX-7). Subsequently, the fraction of Thy1.1-positive CAR T cells with reduced CTV fluorescence intensity through cell division was measured using flow cytometry (FACS Fortessa X-20, BD Bioscience). The results are shown in FIG. 19.

FIG. 15 shows the gene construct structures of the 2^{nd} generation CAR (mCD19BBz) and the 2^{nd} generation CAR and CTLA4-CD28 (mCD19BBz-CTC28) containing the T cell tracking marker (Thy1.1).

FIG. 16 shows graphs analyzing the expression of Thy1.1 and CTC28 proteins on the surface of CAR T cells transduced with mCD19BBz (CD19) or mCD19BBz-CTC28 (CD19-CTC28).

FIG. 17 shows graphs analyzing the tumor killing ability of CAR T cells transduced with mCD19BBz (CD19) or mCD19BBz-CTC28 (CD19-CTC28) against A20 cells.

FIG. 18 illustrates graphs showing the amount of IFN-γ, IL-2, and TNF-α secreted by CAR T cells transduced with mCD19BBz (CD19) or mCD19BBz-CTC28 (CD19-CTC28) when co-cultured with A20 cells.

FIG. 19 shows graphs analyzing the changes in the proliferative capacity of CD8 CAR T cells transduced with mCD19BBz (CD19) or mCD19BBz-CTC28 (CD19-CTC28).

As shown in Figure 16, CD4 T cells transduced with second-generation CAR (mCD19BBz) showed 93.0% Thy1.1-positive cells and 82.2% CTC28-positive cells, while CD8 T cells showed 81.9% Thy1.1-positive cells and 71.8% CTC28-positive cells, confirming good CAR and CTC28 expression in all cases.

As shown in Figure 17, it was confirmed that the tumor killing ability of CD8 CAR T cells, known to lead tumor killing, was higher compared to CD4 CAR T cells. Co-loading of CTC28 did not show significant increases in tumor killing ability in both CD8 and CD4 CAR T cells.

However, as shown in FIG. 18, CTC28 was found to significantly increase the production of cytokines in both CD4 and CD8 T cells.

As shown in FIG. 19, CD8 CAR T cells co-loaded with CTC28 exhibited a more pronounced decrease in CTV staining compared to conventional CD8 CAR T cells, indicating enhanced cytokinesis.

These results confirmed that Thy1.1-labeled CTC28 CAR T cells also showed improved *in vitro* reactivity, and that the reactivity enhancement of CAR T cells prepared by separating CD4 and CD8 T cells was also maintained.

### 3.2. Efficacy of CTC28-loaded CD4 and CD8 CAR T cell combination therapy - in vivo

To evaluate the anti-cancer efficacy of combined administration of CD4 and CD8 CAR T cells *in an in vivo* tumor model of CTC28-loaded second-generation 4-1BB-based CAR T cells, CAR T cells were intravenously injected into Balb/C mice subcutaneously inoculated with A20 cells, and tumor growth was measured over time.

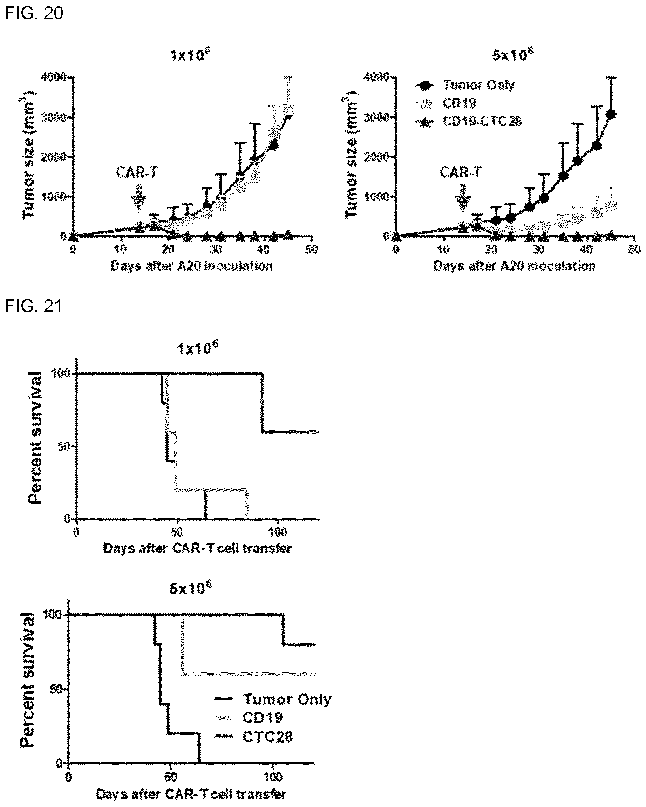

Specifically, BALB/c mice were injected subcutaneously with A20 cells (5X10⁶cells per mouse). Twelve days later, low-dose total body irradiation (2.5 Gy) for peripheral lymphodepletion was performed as a pretreatment prior to CAR T cell infusion. Two days after irradiation, a 1:1 mixture of CD4 CAR T cells and CD8 CAR T cells, expanded 4-5 days after retroviral transduction, were injected intravenously (1X10⁶cells or 5X10⁶cells per mouse). Tumor size was then measured twice a week using a caliper, and the number of CAR T cells in the peripheral blood was measured once a week. Viable CD4 or CD8 CAR T cells in the blood were identified by staining with anti-CD4 antibody (GK1.5, BioLegend), anti-CD8 antibody (53.6.7, BioLegend), anti-Thy1.1 antibody (OX-7, BioLegend), and 7-AAD (BioLegend), followed by CD4(+)Thy1.1(+)7AAD(-) or CD8(+)Thy1.1(+)7AAD(-) cells were counted by flow cytometry using counting beads (123count eBeads, ThermoFisher). The results are shown in FIG. 20 and FIG. 21, respectively.

FIG. 20 illustrates graphs showing the changes in tumor size in mice injected with CAR T cells transduced with mCD19BBz (CD19) or mCD19BBz-CTC28 (CD19-CTC28).

FIG. 21 illustrates graphs showing the survival rate of mice injected with CAR T cells transduced with mCD19BBz (CD19) or mCD19BBz-CTC28 (CD19-CTC28).

As shown in FIG. 20, enhanced tumor suppression activity of second-generation CTC28 CAR T cells compared to 4-1BB-based second-generation CAR T cells was confirmed at both high (5×10⁶) and low (1×10⁶) cell doses. Particularly, even when the efficacy of 4-1BB-based second-generation CAR T cells was barely apparent at low doses (1×10⁶), most tumors were eliminated by second-generation CTC28 CAR T cells.

As shown in FIG. 21, a significant improvement in mouse survival was observed, demonstrating more than 5-fold enhanced therapeutic efficacy by CTC28.

### 3.3. In vivo characterization of CTC28-loaded CD4 and CD8 CAR T cells

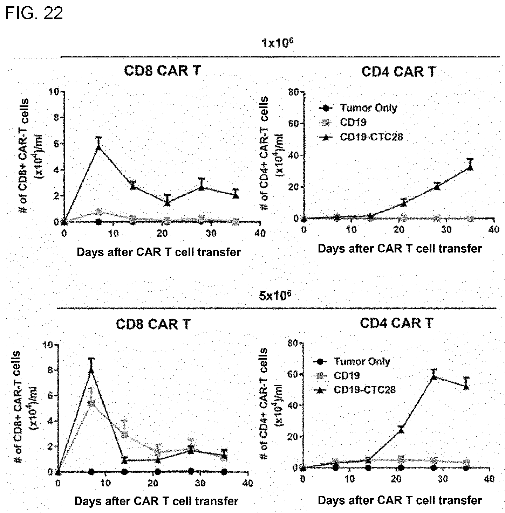

For in vivo follow-up of CD4 and CD8 CAR T cells administered in the CTC28 CD4 and CD8 CAR T cell combination therapy, in a mouse model performed in the same manner as in Example 3.2 above, 35 µl of blood was collected from the tail of the mouse once a week after CAR T cell administration to measure the number of CAR-T cells in the blood, and the results are shown in FIG. 22.

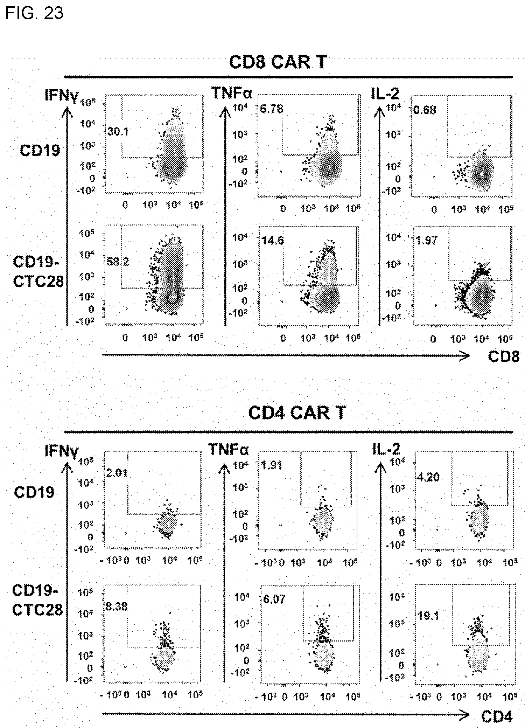

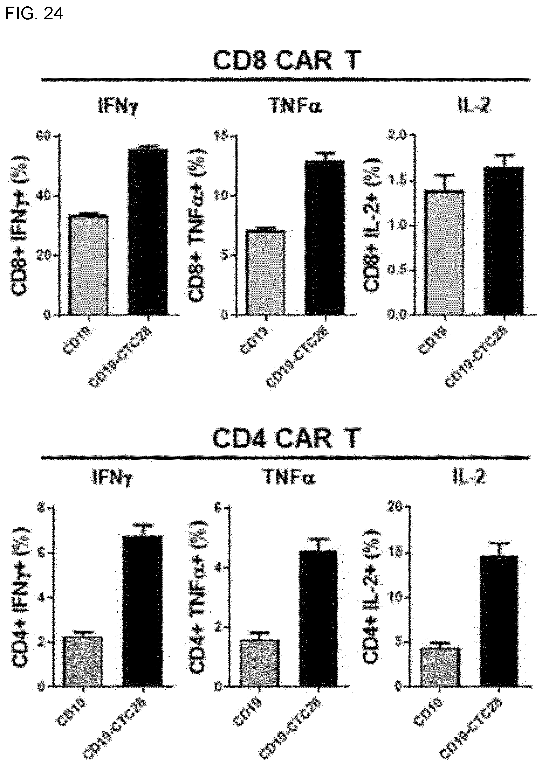

At the peak of CD8 CAR T cell proliferation, on day 7, CAR T cells isolated from mouse spleen were co-cultured with A20 cells for 6 hours, and the INF-γ, IL-2, and TNF-α secreted by CD4 and CD8 CAR T cells were analyzed by flow cytometry plots (ex vivo). The results are shown in FIG. 23 and the graphs quantifying them are shown in FIG. 24.

FIG. 22 indicates graphs showing the numbers of CD4 and CD8 CAR T cells transduced with mCD19BBz (CD19) or mCD19BBz-CTC28 (CD19-CTC28) in the A20 cell tumor mouse model.

FIG. 23 illustrates graphs showing the amounts of IFN-γ, IL-2, and TNF-α secreted by CD4 and CD8 CAR T cells transduced with mCD19BBz (CD19) or mCD19BBz-CTC28 (CD19-CTC28) in the A20 cell tumor mouse model.

FIG. 24 illustrates graphs quantifying the amounts of IFN-γ, IL-2, and TNF-α secreted by CD4 and CD8 CAR T cells transduced with mCD19BBz (CD19) or mCD19BBz-CTC28 (CD19-CTC28) in the A20 cell tumor mouse model.

As shown in FIG. 22, a significant increase in proliferation of CTC28-loaded CAR T cells was observed in both CD4 and CD8 CAR T cells compared to conventional CAR T cells. CTC28-loaded CD8 CAR T cells exhibited dramatic initial proliferation in the blood, whereas CTC28-loaded CD4 CAR T cells were characterized by later proliferation, ultimately showing much greater cell proliferation compared to CD8 CAR T cells, demonstrating CD4 bias.

As shown in FIGS. 23 and 24, CTC28-transduced CD8 CAR T cells and CD4 CAR T cells were found to be highly functionally active, as both were observed to be cytokine-secreting at day 7.

### 3.4. Antitumor mechanism of CTC28-loaded CD4 and CD8 CAR T cells

In a previous study on classical T cell therapy loaded with CTC28, CD8 T cell activation by CD4 T cells (so-called CD4 help) was greatly enhanced when CTC28 was introduced into CD4 T cells (Blood 2012;119(24):5678-87). To confirm whether enhancement of CD4 help by CTC28 contributes to antitumor effect enhancement in this CAR T cell situation, CTC28-loaded CD4 CAR T cells were co-administered with CD8 CAR T cells not loaded with CTC28.

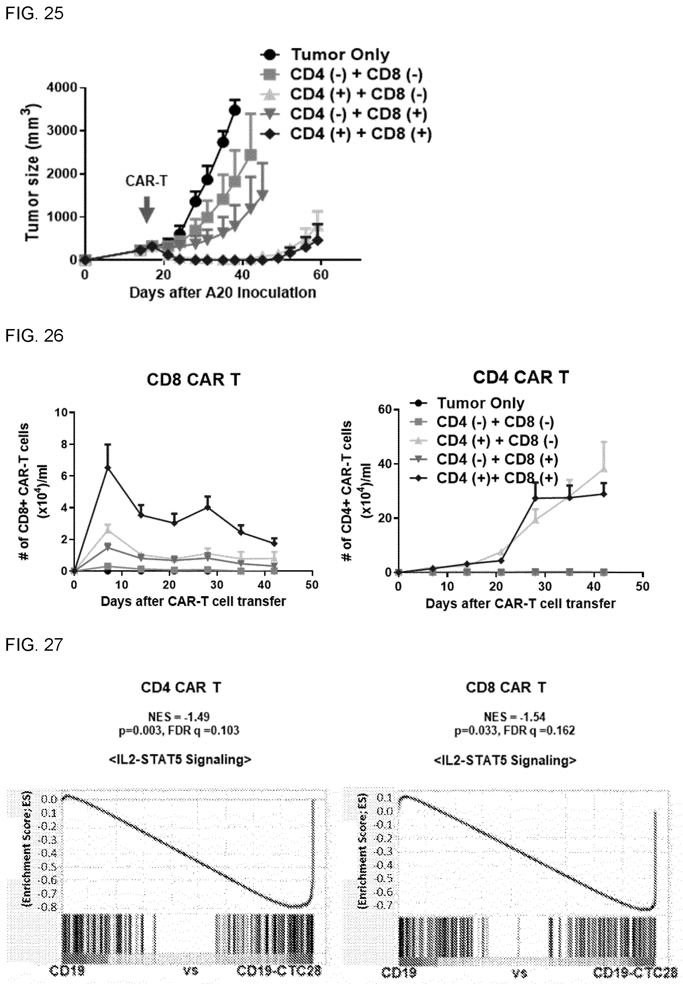

Specifically, Balb/C mice were injected subcutaneously with A20 cells (^{5X10(6)}cells per mouse) in the same manner as in Example 3.2 above, and CD4 and CD8 CAR T cells were injected intravenously (^{1X10(6)}cells per mouse) after irradiation, and the tumor growth and the number of each CD4 and CD8 CAR T cells were measured. The results are shown in FIGS. 25 and 26, respectively.

FIG. 25 illustrates a graph showing the changes in tumor size in mice co-administered with a 1:1 mixture of CD4 or CD8 mCD19BBz CAR T cells with (+) or without (-) CTC28 in the A20 cell tumor mouse model (Tumor size = (long axis)² x short axis/2).

FIG. 26 indicates graphs showing the numbers of CAR T cells in mice co-administered with a 1:1 mixture of CD4 or CD8 mCD19BBz CAR T cells with (+) or without (-) CTC28 in the A20 cell tumor mouse model.

As shown in FIG. 25, CTC28 alone on CD4 CAR T cells significantly boosted the antitumor potency of CD8 CAR T cells without CTC28 (CD4(-) + CD8(-) vs CD4(+) + CD8(-)), confirming that CD4-help is significantly increased by CTC28. However, a modest increase in antitumor potency was also observed when CTC28 was expressed on CD8 CAR T cells alone (CD4(-) + CD8(-) vs CD4(-) + CD8(+)), suggesting that CTC28 also increases the intrinsic activity of CD8 CAR T cells.

In terms of cell proliferation, as shown in FIG. 26, CTC28 transfection of CD4 CAR T cells alone significantly increased the initial proliferation of CD8+ CAR T cells compared to CD4 CAR T cells without CTC28 (CD4(-) + CD8(-) vs CD4(+) + CD8(-)), confirming that CTC28 on CD4 CAR T cells enhances CD4 helper function on CD8 CAR T cells. However, direct proliferation of CTC28 on CD8 CAR T cells was also observed (CD4(-) + CD8(-) vs CD4(-) + CD8(+)), with the greatest proliferation of CD8 CAR T cells observed when CTC28 was introduced to both CD4 and CD8 CAR T cells (CD4(+) + CD8(+)), suggesting that introduction of CTC28 to both CD4 and CD8 CAR T cells is most effective,

These results confirm that CTC28 is able to enhance the function of both CD4 and CD8 CAR T cell populations.

To track the factors mediating the enhancement of CAR T cell function by CTC28, CD4 and CD8 CAR T cells were isolated from the spleen of mice on day 4 after CAR T cell infusion and RNA sequencing was performed by gene set enrichment analysis (GSEA).

Specifically, in Example 3.2 above, A20 cells were injected subcutaneously and low-dose total body irradiation was performed on day 14 when tumors reached ~200 mm^{(3),}followed by intravenous infusion of 5x10⁶ CAR T cells without CTC28 (mCD19BBz(CD19)) (CD4:CD8 = 1:1) or CAR T cells with CTC28 (mCD19BBz-CTC28(CD19-CTC28)) (CD4:CD8 = 1:1), respectively. Four days after CAR T cell infusion, spleens were harvested from mice and probed in single cell suspension with anti-CD4 antibody (GK1.5), anti-CD8 antibody (53.6.7), anti-Thy1.1 antibody (OX-7), and stained with DAPI (Thermofisher). CD4(+)Thy1.1(+)DAPI(-) or CD8(+)Thy1.1(+)DAPI(-) cells in the spleen were isolated and purified using a cell sorter. RNA from CAR T cells was extracted with the RNeasy Mini kit (Qiagen), and RNA sequencing was performed by Macrogen. Gene set enrichment analysis (GSEA) was performed using GSEA software (gsea-v4.3.0, http://software.broadi nstitute.org/gsea/downinfects.jsp). RNA sequencing data from two replicates were subjected to GSEA using Combat-seq (Bioconductior sva v3.36.0, https://bioconductor.org/packages/release/bioc/html/ sva.html) after correction for batch effects between the two samples. The p-value was calculated by Kolmogorov-Smirnov test (threshold = 0.01), and the FDR (False Discovery Rate)-q value is the estimated probability of a false positive result for a gene set with a given NES. The threshold for q-value in GSEA is 0.25, and the Hallmark gene set was used for GSEA analysis. The results are shown in FIG. 27.

As shown in FIG. 27, the analysis showed that RNAs involved in IL-2-STAT5 signaling were enriched in CTC28 CD4 and CD8 CAR T cells. This suggested that IL-2 may have contributed to the proliferation of CTC28 CAR T cells.

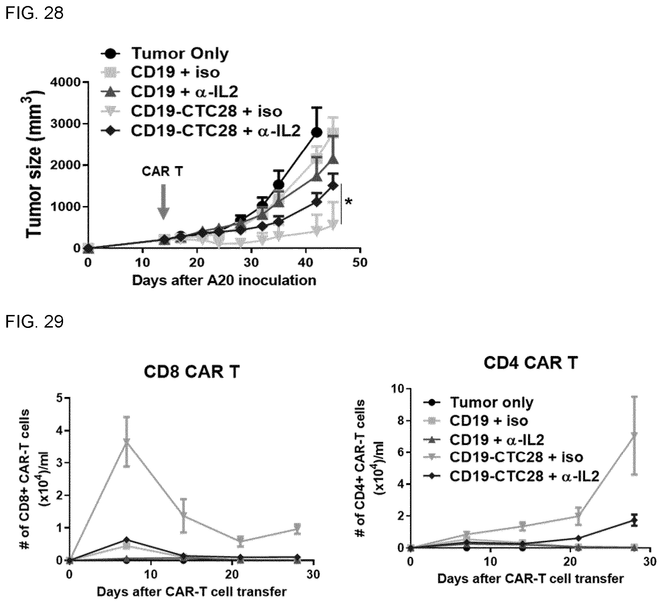

To determine whether IL-2 mediates CTC28 CAR T cell proliferation, specifically, Balb/C mice were injected subcutaneously with A20 cells (5X10⁶ cells per mouse) in the same manner as in Example 3.2, followed by irradiation and intravenous infusion of CD4 and CD8 CAR T cells (1X10⁶ cells per mouse). IL-2 neutralization antibody (α-IL-2:JES6-1A12, BioXcell) or isotype control antibody (iso:Rat IgG2a, BioXcell) was then injected intraperitoneally at 200 µg every 2-3 days starting from the time of CAR T cell injection. Tumor size and the number of each CD4 and CD8 CAR T cells were measured and the results are shown in FIGS. 28 and 29, respectively.

FIG. 27 illustrates graphs showing the RNA-seq analysis of CD4 and CD8 CAR T cells transduced with mCD19BBz (CD19) or mCD19BBz-CTC28 (CD19-CTC28) in the A20 cell tumor mouse model.

FIG. 28 illustrates a graph showing the changes in tumor size in mice treated with CAR T cells transduced with mCD19BBz (CD19) or mCD19BBz-CTC28 (CD19-CTC28) together with an IL-2 neutralizing antibody (α-IL2) in the A20 cell tumor mouse model (Tumor size = (long axis)² x short axis/2).

FIG. 29 indicates graphs showing the numbers of CAR T cells in mice treated with CAR T cells transduced with mCD19BBz (CD19) or mCD19BBz-CTC28 (CD19-CTC28) together with an IL-2 neutralizing antibody (α-IL2) in the A20 cell tumor mouse model.

As shown in FIGS. 28 and 29, both the tumor suppressive ability of CTC28-mediated CAR T cells and their proliferative capacity in blood were decreased in the IL-2 neutralizing antibody-treated group, indicating that IL-2 contributes to the enhancement of CTC28-mediated CAR T cell function.

Based on the above results, the mechanism of CAR T cell functional enhancement of CTC28 is due to the enhancement of CD4 help and CD8 T cells, and IL-2 contributes partially to the functional enhancement.

### Example 4. Safety of CTC28 CAR T cells

The characteristic of CTC28-loaded CAR T cells in blood from the above experimental examples is a significant increase in cell numbers. To verify the safety of this cell number increase, the increase and decrease of CAR T cell numbers in peripheral blood were tracked long-term for about 5 months after CAR T cell administration. Additionally, mouse weight loss was tracked as a toxicity indicator.

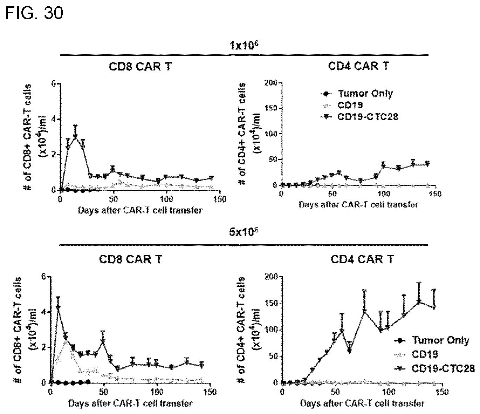

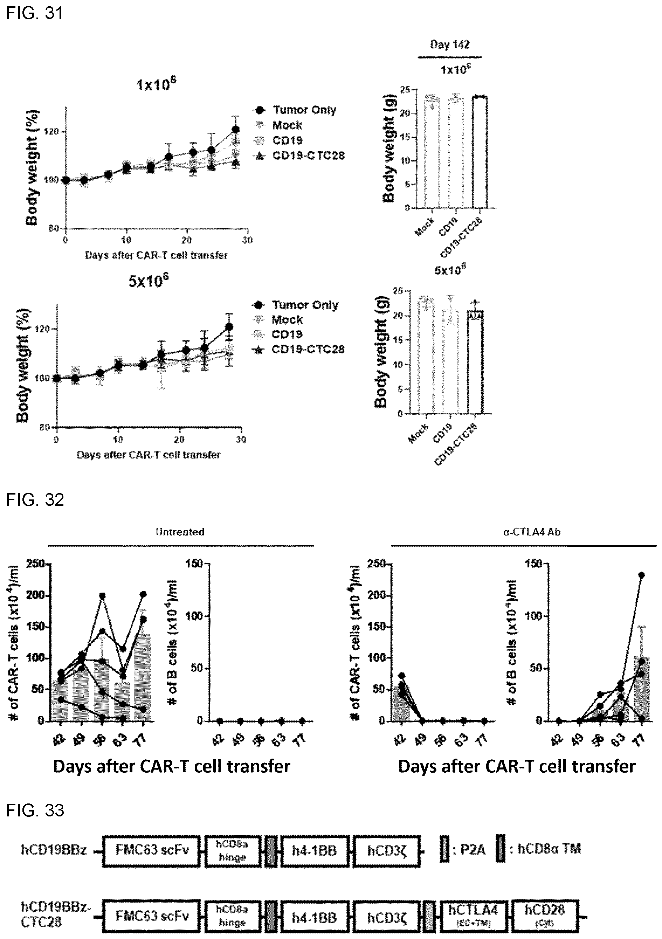

Specifically, Balb/C mice were injected subcutaneously with A20 cells (5X10⁶ cells per mouse) in the same manner as in Example 3.2 above, followed by irradiation and intravenous injection of CAR T cells (1X10⁶ or 5X10⁸ cells per mouse), and 35 µl of blood was collected from the tail of the mice once a week to measure the number of CAR-T cells in the blood. To evaluate the toxicity of CAR-T, the weight loss and survival of each mouse was continuously monitored twice a week after CAR T infusion, and the weight of the mice was measured for 28 days after CAR T cell administration and 142 days after administration. The results are shown in FIGS. 30 and 31.

FIG. 30 indicates graphs showing the numbers of CD4 and CD8 CAR T cells in the peripheral blood of mice after administration of CAR T cells transduced with mCD19BBz (CD19) or mCD19BBz-CTC28 (CD19-CTC28) in the mouse model.

FIG. 31 illustrates graphs showing the changes in body weight of mice after administration of CAR T cells transduced with mCD19BBz (CD19) or mCD19BBz-CTC28 (CD19-CTC28) in the mouse model.

As shown in FIG. 30, CD8 CTC28 CAR T cells were found to consistently maintain a stable cell count at a higher level than normal CAR T cells, although the number of cells decreased again after the initial mass proliferation. CD4 CTC28 CAR T cells, which proliferate more rapidly in the second half, show a sustained increase in cell number after about 3 weeks, but maintain a stable cell number after about 2 months.

As shown in FIG. 31, there was no weight loss in the CTC28 CAR T cell group in the first 1-2 weeks, which could be indicative of acute toxicity, and the weight measured after 5 months was not significantly different compared to the classical CAR T cell group. This suggests that CTC28-loaded CAR T cells are highly unlikely to exhibit acute and chronic toxicity.

Nevertheless, if chronic toxicity due to large numbers of CTC28-loaded CAR T cells present in the long term is a concern, the ability to eliminate these CTC28-loaded CAR T cells would provide a safety assurance. As part of this, it has been reported that the currently marketed α-CTLA4 antibody (Ipilimumab) has the ability to eliminate CTLA4-expressing cells. Therefore, if the α-CTLA4 antibody binds to the extracellular CTLA4 site on CTC28, it may act to eliminate CTC28-loaded CAR T cells. In this case, CTC28 could act as a pro- and anti-apoptotic receptor as well as a suicide receptor.

Therefore, it was determined whether the increased CTC28 CAR T cells in the blood could be eliminated using the α-CTLA4 antibody. Specifically, A20 cells (X10(6) per mouse) were injected into Balb/C mice in the same manner as in Example 3.2 above, Balb/C mice were injected subcutaneously with A20 cells (5X10⁶ cells per mouse), followed by irradiation and intravenous injection of CAR T cells (5X10⁶ cells per mouse), and 42 days after CAR T cell administration, 200 µg of α-CTLA4 antibody (UC10-4B9, BioXcell) was injected intraperitoneally, followed by three more injections of 100 µg of α-CTLA4 antibody at 2-day intervals, and the number of Thy1.1-positive CAR T cells and CD19-positive B cells in peripheral blood were tracked by flow cytometry by collecting 35 µl of blood from the mouse tail once a week. The results are shown in FIG. 32.

FIG. 32 illustrates graphs showing the numbers of CAR T cells and B cells in the blood of mice after administration of CAR T cells transduced with mCD19BBz (CD19) or mCD19BBz-CTC28 (CD19-CTC28) and treatment with an anti-CTLA4 antibody in the mouse model.

As shown in FIG. 32, all CTC28-loaded CAR T cells in blood were eliminated when treated with α-CTLA4 antibody. CD19-targeting CAR T cells are known to have the side effect of eliminating CD19-positive tumors as well as CD19-positive normal B cells in the blood. Therefore, no B cells were detected in the blood in the presence of CTC28-loaded CAR T cells (untreated group). However, after the CAR T cells were eliminated by α-CTLA4 antibody treatment, B cells were gradually detected in the blood, indicating that the CAR T cells were completely eliminated. This suggests the possibility of administering a commercially available α-CTLA4 antibody (Ipilimumab) to eliminate CTC28-loaded CAR T cells, if necessary, when actual CTC28-loaded CAR T cells are administered in the clinic.

These results confirm the safety of CTC28-loaded CAR T cell therapy by demonstrating that CTC28 CAR T cells that accumulate in the blood over the long term do not exhibit toxicity and can be eliminated with anti-CTLA4 antibodies when necessary.

### Example 5. Anti-cancer efficacy of human CTC28-loaded CAR T cells

### 5.1. Construction of human CTC28-loaded CAR lentivirus and T cell transduction

Human CTC28-loaded CAR T cells were prepared to confirm the effect of CTC28 on CAR T cell function in human CAR T cells.

Specifically, the protein coding region cDNA of 4-1BB-based CAR (hCD19BBz) using human anti-CD19 antibody (FMC63) was synthesized according to previously published sequences (US Patent Publication No. US 2013/0287748 A1) by commissioning DNA synthesis from IDT USA. The lentiviral vector for human CAR expression was constructed by cloning human CAR cDNA into a partially modified pCDH-EF1 vector (Addgene Plasmid #72266). The human CTC28 protein coding region was designed as a form linking corresponding regions of human CTLA4 and CD28, and was constructed by commissioning DNA synthesis from IDT after human codon optimization of the DNA sequence. The lentiviral vector for CAR and CTC28 dual expression was constructed by cloning human CTC28 cDNA linked with P2A sequence downstream of the human CAR protein coding region. The schematic is shown in FIG. 33.

For the production of human CAR expression lentivirus, each lentiviral vector was transfected into 293T cell line (ATCC, USA) using Lipofectamine 3000 (Invitrogen) together with three types of packaging DNA (pMD.2G; pMDLg/pRRE, addgene plasmid #12251; pRSV-rev, addgene plasmid 12253). After 24-48 hours, the culture supernatant containing secreted lentivirus was harvested and filtered (0.45µm filter) to remove residual cell particles, concentrated 100-fold using an ultracentrifuge, and used as lentiviral concentrate for human CAR T cell production.

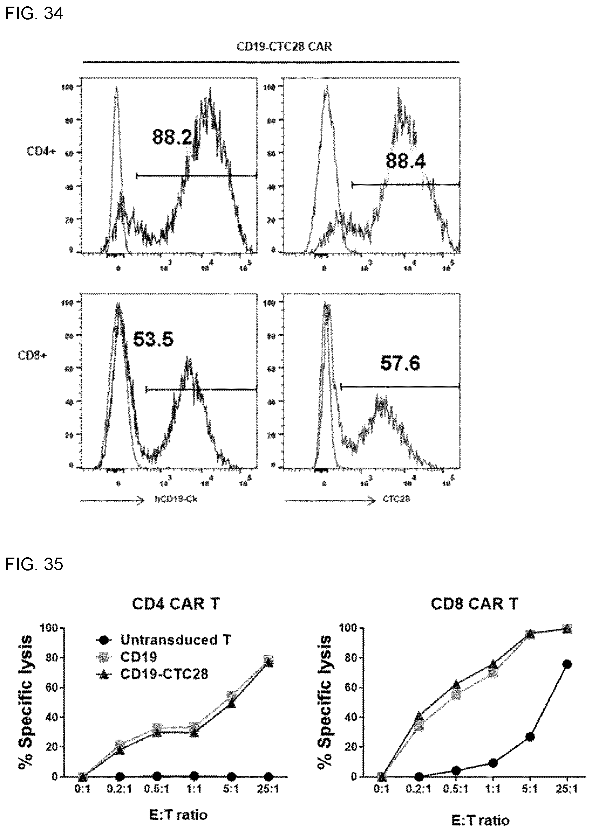

For human CAR T cells, leukocytes obtained by leukapheresis from normal individuals were stained with anti-CD4 microbeads or anti-CD8 microbeads (Miltenyi Biotech), and CD4 T cells and CD8 T cells were separated using MACS LS columns (Miltenyi Biotech). The isolated T cells were added to 24 well plates coated with anti-CD3 antibody (OKT3, 10/µgmℓ, BioXcell) together with anti-CD28 antibody (CD28.2, 2/µgmℓ, BD Biosciences) and incubated for 48 hours to activate the T cells. Activated T cells were washed twice and used for lentiviral transduction. Retronectin (20/µgmℓ, TaKaRa) was coated overnight at 4°C and then washed 24 well plates were added to 2% BSA-DPBS, blocked and washed at 37°C for 30 minutes, lentiviral concentrate was added and centrifuged at 2000xg, 32°C for 2 hours to attach the lentivirus to the bottom of the wells. After removing the viral concentrate and washing the wells, 1 ml of activated T cells (1X10⁶ cells/mℓ) was added to the wells and centrifuged for 10 min (1000xg, 32°C) to attach the cells to the lentivirus. The cells were then incubated in the presence of human IL-2 (300IU/mℓ, Proleukin, Novartis) for 48 hours. The lentiviral-transduced T cells were washed twice, and then expanded for 3-6 days in fresh culture medium containing human IL-2 (200IU/mℓ) to be used as CAR T cells. The expression of CAR proteins on the cell surface was measured by flow cytometry (FACS-Canto II, BD Biosciences) after staining CAR T cells proliferated for 3-6 days after lentiviral transduction with CD19-Ck protein (a fusion protein of a self-made CD19 extracellular region and human immunoglobulin kappa chain invariant (Ck)) and anti-Ck antibody labeled with APC (anti-Ck-APC, BioLegend). The expression of CTC28 was on the cell surface was confirmed by staining with fluorescently labeled anti-CTLA4 antibody (L3D10, BioLegend). The results are shown in FIG. 34.

FIG. 33 shows the gene construct structures of the human 41BB-based CAR (hCD19BBz) and the human CTC28 co-expressed with the CAR (hCD19BBz-CTC28).

FIG. 34 shows graphs analyzing the expression of CAR and CTC28 proteins on the surface of T cells transduced with the human CAR (hCD19BBz) or the human CTC28 (CD19-CTC28 CAR).

As shown in Figure 34, human CD19-CTC28 CD4 T cells showed 88.2% hCD19-Ck positive cells and 88.4% CTC28 positive cells, while human CD19-CTC28 CD8 T cells showed 53.5% hCD19-Ck positive cells and 57.6% CTC28 positive cells, confirming good CAR and CTC28 expression.

### 5.2. Anti-cancer efficacy of human CTC28-loaded CAR T cells - in vitro

To evaluate *the in vitro* anti-cancer efficacy of human CTC28-loaded CAR T cells, the tumor killing ability of the produced human CAR T cells against Raji cells, a human tumor cell line, was analyzed.

Specifically, Raji cells (human B cell lymphoma cell line) were purchased from ATCC (USA), and the Raji cell line with introduced GFP-luciferase gene (Raji-Luc) was prepared by transducing Raji cells with GFP-Luc expression lentivirus (pLEF-LucGFP) and then isolating GFP-positive cells using a cell sorter (FACS Aria, Becton Dickinson). For measuring the tumor killing ability of CAR T cells, CAR T cells proliferated for 3 days after CAR lentiviral transduction (Effector cell, 1.2×10³ to 7.5×10⁵ cells/100µl/well) were added to Raji-Luc cells (Target cell, 3×10⁴ cells/50µl/well) at various ratios (0.04 to 25:1) and co-cultured overnight in 96 well plates. Then, D-Luciferin (600µg/ml, Promega) 50µl was added and incubated at 37°C for 10 minutes to induce luciferase enzyme activity in surviving Raji-Luc cells. The luminescence of these cells was measured using a Luminometer (Tecan), and the tumor killing ability of CAR T cells was measured by calculating the survival rate of tumor cells compared to the luminescence of Raji-Luc cells not treated with CAR T cells. The results are shown in FIG. 35.

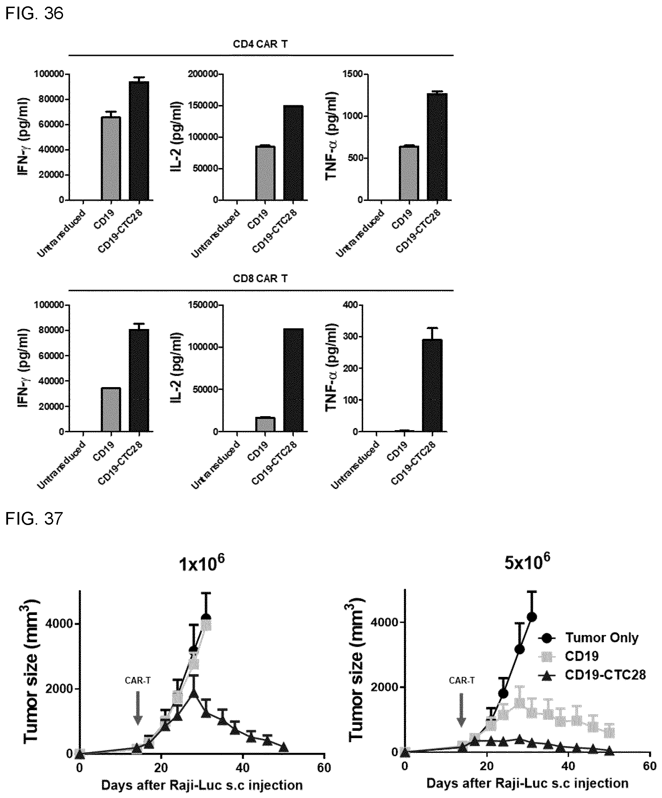

To evaluate the cytokine production ability of CAR-transduced CD4 and CD8 T cells, the amounts of IFN-γ, IL-2, and TNF-α secreted when CAR-transduced CD4 and CD8 T cells were co-cultured with Raji cells were measured by the same method as in Example 3.1 above, and the results are shown in FIG. 36.

FIG. 35 shows graphs analyzing the tumor killing ability of T cells transduced with the human hCD19BBz (CD19) or hCD19BBz-CTC28 (CD19-CTC28) against Raji cells.

FIG. 36 illustrates graphs showing the amounts of IFN-γ, IL-2, and TNF-α secreted by T cells transduced with the human hCD19BBz (CD19) or hCD19BBz-CTC28 (CD19-CTC28) when co-cultured with Raji cells.

As shown in FIG. 35, the increase in tumor killing ability of CAR T cells by human CTC28 was not significantly observed for both CD4 and CD8 CAR T cells.

However, as shown in FIG. 36, the cytokine production ability of human CD4 and CD8 CAR T cells was significantly increased by CTC28 transduction.

### 5.3. Anti-cancer efficacy of human CTC28-loaded CAR T cells - in vivo

The *in vivo* efficacy of human CAR T cells is usually evaluated by inoculating human tumor cells into immunodeficient mice (e.g., NSG mice) and then administering human CAR T cells to see that tumor growth is inhibited.

Therefore, to evaluate the *in vivo* anticancer efficacy of combined administration of human CTC28-loaded CD4 and CD8 CAR T cells, CAR T cells were intravenously injected into NSG mice subcutaneously inoculated with Raji cells, and changes in tumor growth and the numbers of CD4 and CD8 CAR T cells in blood were measured over time.

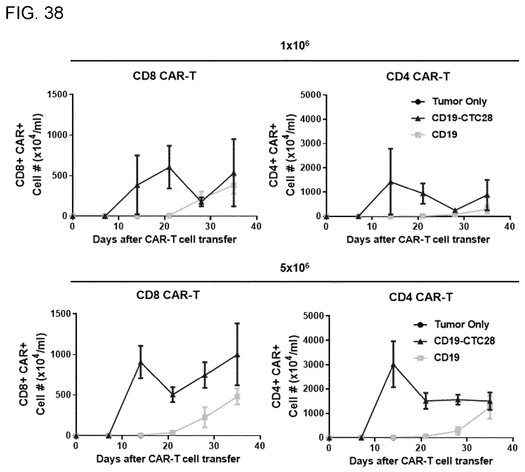

Specifically, NSG mice were injected subcutaneously with Raji-Luc cells (5X10⁵ cells per mouse) and 7 days later, intravenously with a 1:1 mixture of expanded CD4 CAR T cells and CD8 CAR T cells (1X10⁶cells or 5X10⁶cells per mouse) 7-10 days after CAR lentiviral transduction. Tumor size was then measured twice a week using a caliper, and the number of CAR T cells in the peripheral blood was measured once a week. Viable CD4 or CD8 CAR T cells in the blood were detected using anti-CD3ε antibody (UCHT1, BioLegend), anti-CD4 antibody (SK3, BioLegend), anti-CD8 antibody (SK1, BioLegend), anti-FMC63 scFv antibody (FM3-HPY53, Acrobio), After staining with 7-AAD (BioLegend), the number of CD3(+)CD4(+)CAR(+)7AAD(-) or CD3(+)CD8(+)CAR(+)7AAD(-) cells was counted by flow cytometry using contingent beads (123count eBeads, ThermoFisher). The respective results are shown in FIGS. 37 and 38.

FIG. 37 illustrates graphs showing the changes in tumor size in mice injected with T cells transduced with the human hCD19BBz (CD19) or hCD19BBz-CTC28 (CD19-CTC28) (Tumor size = (long axis)² x short axis/2).

FIG. 38 illustrates graphs showing the numbers of CD4 and CD8 CAR T cells in NSG immunodeficient mice injected with the human hCD19BBz (CD19) or hCD19BBz-CTC28 (CD19-CTC28).

As shown in FIG. 37, it was found that CTC28-loaded CAR T cells exhibited significant tumor suppression compared to conventional CAR T cells.

A significant increase in the number of CTC28 CAR T cells in the blood was also observed, as shown in FIG. 38.

This study demonstrated the ability of CTC28 to promote CAR T cell function in the human CAR T cell system, confirming its applicability as a future CAR T cell therapy with enhanced function.

Based on the above results, CTC28 CAR T cell therapy may contribute significantly to improving the efficacy of CAR T cell therapies by showing more advanced CAR T cell function compared to conventional CAR T cell therapies.

The foregoing description of the invention is for illustrative purposes only, and those having ordinary knowledge in the technical field to which the invention belongs will understand that it can be readily adapted to other specific forms without altering the technical idea or essential features of the invention. The embodiments described above are therefore to be understood in all respects as exemplary and not limiting.

## Claims

1. A construct comprising:
(i) a first gene encoding a fusion protein (CTLA4-CD28 fusion protein) comprising a CTLA4 (Cytotoxic T Lymphocyte Antigen-4) protein or a domain thereof; and CD28 or a domain thereof; and
(ii) a second gene encoding a chimeric antigen receptor (CAR) comprising an antigen binding domain; an extracellular spacer domain; a transmembrane domain; and an intracellular signaling domain.

2. The construct of claim 1, wherein the CTLA4-CD28 fusion protein comprises a CTLA4 extracellular domain and a CD28 intracellular domain.

3. The construct of claim 1 or 2, wherein the CTLA4-CD28 fusion protein comprises either a CTLA4 extracellular domain-CTLA4 transmembrane domain-CD28 intracellular domain, or a CTLA4 extracellular domain-CD28 transmembrane domain-CD28 intracellular domain.

4. The construct of any one of claims 1 to 3, wherein CTLA4 comprises the amino acid sequence of SEQ ID NO: 1 or SEQ ID NO: 2; or an amino acid sequence having at least 90% identity thereto.

5. The construct of any one of claims 1 to 4, wherein CD28 comprises the amino acid sequence of SEQ ID NO: 3 or SEQ ID NO: 4; or an amino acid sequence having at least 90% identity thereto.

6. The construct of any one of claims 3 to 5, wherein the CTLA4 extracellular domain-CTLA4 transmembrane domain-CD28 intracellular domain comprises the amino acid sequence of SEQ ID NO: 5 or SEQ ID NO: 6; or an amino acid sequence having at least 90% identity thereto.

7. The construct of any one of claims 3 to 5, wherein the CTLA4 extracellular domain-CD28 transmembrane domain-CD28 intracellular domain comprises the amino acid sequence of SEQ ID NO: 7; or an amino acid sequence having at least 90% identity thereto.

8. The construct of any one of claims 1 to 7, wherein the chimeric antigen receptor is either (a) a chimeric antigen receptor comprising an antigen binding domain; an extracellular spacer domain; a transmembrane domain; and a CD3zeta intracellular signaling domain, or (b) a chimeric antigen receptor comprising an antigen binding domain; an extracellular spacer domain; a transmembrane domain; a 4-1BB intracellular domain; and a CD3zeta intracellular signaling domain.

9. The construct of claim 8, wherein the chimeric antigen receptor comprises the amino acid sequence of SEQ ID NO: 9, SEQ ID NO: 13, or SEQ ID NO: 15; or an amino acid sequence having at least 90% identity thereto.

10. The construct of any one of claims 1 to 9, wherein the antigen binding domain comprises an antibody or antigen-binding fragment thereof that specifically binds to one or more antigens selected from the group consisting of:
4-1BB, BCMA, BAFF, B7-H3, B7-H6, CA9, CTAG1B, CEA, cyclin, cyclin A2, cyclin B1, CCL-1, CCR4, CD3, CD4, CD19, CD20, CD22, CD23, CD24, CD30, CD33, CD38, CD40, CD44, CD44v6, CD44v7/8, CD52, CD58, CD62, CD79A, CD79B, CD80, CD123, CD133, CD138, CD171, CSPG4, CLDN18, CLDN18.2, CLDN6, CTLA-4, c-Met, DLL3, EGFR, tEGFR, EGFRvIII, EPG-2, EPG-40, ephrin B2, EPHA2, estrogen receptor, Fc receptor, FCRL5, FGF23, FBP, FOLR1, FOLR2, GD2, ganglioside GD3, gp100, GPC3, GPCR5D, GM-CSF, Her2/neu, Her3, Her4, erbB dimers, HMW-MAA, HBsAg, HLA-A1, HLA-A2, IL-22Ra, IL-13Ra2, ICOS, IGF-1 receptor, integrin αvβ6, interferon receptor, IFNγ, IL-2R, IL-4R, IL-5R, IL-6R, IL-17RA, IL-31R, IL-36R, kdr, L1-CAM, CE7 epitope of L1-CAM, LRRC8A, Lewis Y, LAG3, MAGEA1, MAGEA3, MAGEA6, MAGEA10, MSLN, CMV, MUC1, NKG2D ligand, MART-1, NGF, NCAM, NRP-1, NRP-2, carcinoembryonic antigen, PD-L1, PRAME, progesterone receptor, prostate-specific antigen, PSCA, PSMA, RANKL, ROR1, SLAMF7, survivin, TPBG, TAG72, TRP1, TRP2, and Wilms tumor 1 (WT1).

11. The construct of any one of claims 1 to 10, wherein the antigen binding domain is an antibody, an antigen-binding fragment thereof, a ligand protein binding to an antigen, or a domain thereof, wherein the antigen-binding fragment is a single chain variable fragment (scFv), (scFv)₂, Fv, Fab, Fab', F(ab')₂ or nanobody of an antibody, or a combination thereof.

12. The construct of any one of claims 1 to 11, wherein the chimeric antigen receptor further comprises a signal peptide at the N-terminus of the antigen binding domain.

13. The construct of any one of claims 1 to 12, wherein the first gene encoding the CTLA4-CD28 fusion protein and the second gene encoding the chimeric antigen receptor are linked to each other by a 2A peptide sequence or an IRES sequence, or wherein the first gene and the second gene are each operably linked to independent promoters.

14. The construct of any one of claims 1 to 13, wherein the construct comprises the amino acid sequence of SEQ ID NO: 17, SEQ ID NO: 21, or SEQ ID NO: 23; or an amino acid sequence having at least 90% identity thereto.

15. A vector comprising the construct according to any one of claims 1 to 14.

16. The vector of claim 15, wherein the vector is a viral vector or a non-viral vector.

17. The vector of claim 16, wherein the viral vector is selected from the group consisting of retrovirus, lentivirus, adenovirus, adeno-associated virus, and Vaccinia virus.

18. An immune cell into which the construct according to any one of claims 1 to 14 or a vector comprising the construct has been introduced.

19. An immune cell comprising a protein expressed from the construct according to any one of claims 1 to 14 or a vector comprising the construct.

20. An immune cell comprising:
(i) a first gene encoding a fusion protein (CTLA4-CD28 fusion protein) comprising a CTLA4 protein or a domain thereof; and CD28 or a domain thereof; and
(ii) a second gene encoding a chimeric antigen receptor comprising an antigen binding domain; an extracellular spacer domain; a transmembrane domain; and an intracellular signaling domain.

21. An immune cell expressing both:
(i) a fusion protein (CTLA4-CD28 fusion protein) comprising a CTLA4 protein or a domain thereof; and CD28 or a domain thereof; and
(ii) a chimeric antigen receptor comprising an antigen binding domain; an extracellular spacer domain; a transmembrane domain; and an intracellular signaling domain.

22. The immune cell of any one of claims 18 to 21, wherein the immune cell is a T cell, NK cell, NKT cell, macrophage, or a combination thereof.

23. The immune cell of any one of claims 18 to 22, wherein the immune cell is a CD4 T cell, CD8 T cell, or a combination thereof.

24. A composition comprising: the construct according to any one of claims 1 to 14; a vector comprising the construct; an immune cell into which the construct or the vector has been introduced; or the immune cell according to any one of claims 18 to 23.

25. A pharmaceutical composition for the treatment of cancer, comprising: the construct of any one of claims 1 to 14; a vector comprising the construct; an immune cell into which the construct or the vector has been introduced; or the immune cell of any one of claims 18 to 23, and a pharmaceutically acceptable carrier.

26. A method for producing an immune cell expressing (a) a fusion protein (CTLA4-CD28 fusion protein) comprising a CTLA4 protein or a domain thereof; and CD28 or a domain thereof and (b) a chimeric antigen receptor comprising an antigen binding domain; an extracellular spacer domain; a transmembrane domain; and an intracellular signaling domain, the method comprising the step of introducing into an immune cell:
(i) a first gene encoding the CTLA4-CD28 fusion protein; and
(ii) a second gene encoding the chimeric antigen receptor.

27. The method of claim 26, wherein the step comprises introducing into the immune cell the construct according to any one of claims 1 to 14, or a vector comprising the construct.
